# EUROPEAN PATENT SPECIFICATION

(11) **EP 3 211 402 B1**
(45) Date of publication and mention of the grant of the patent: **11.07.2018**
(21) Application number: 17165496.5
(22) Date of filing: 28.11.2012
(51) Int. Cl.: G01N 21/64, A61M 1/16, C02F 1/00, C02F 1/50, G01N 15/14, C02F 1/44

(54) **VIABLE PARTICLE COUNTER AND DIALYSIS FLUID MONITORING SYSTEM**
ZÄHLER FÜR LEBENDPARTIKEL UND SYSTEM ZUR ÜBERWACHUNG VON DIALYSEFLÜSSIGKEIT
COMPTEUR DE PARTICULES VIABLES ET SYSTÈME DE SURVEILLANCE DE FLUIDE DE DIALYSE

(30) Priority: 05.12.2011 JP 2011265613; 16.01.2012 JP 2012006107; 17.01.2012 JP 2012007338
(43) Date of publication of application: 30.08.2017
(62) Divisional of application: 12855154.6
(73) Proprietor: Rion Co., Ltd., Tokyo 185-8533 (JP)
(72) Inventor: ICHIJYO, Kazuo, 185-8533 Tokyo (JP); SEKIMOTO, Kazuma, Tokyo, 185-8533 (JP); SEKIGAWA, Kousei, 185-8533 Tokyo (JP); KIMOTO, Yukihiro, Tokyo, 185-8533 (JP); KOSAKA, Takayuki, Tokyo, 185-8533 (JP)
(74) Representative: Isarpatent

(56) References cited:
- EP-A1- 2 218 472
- WO-A1-90/14589
- WO-A1-2010/024963

## Description

### [Technical Field]

The present invention relates to a viable particle counter which detects viable particles in a dialysis fluid in real time and a dialysis fluid monitoring system.

### [Background Art]

In the detection of viable particles, there have been known a method of cultivation (official analytical method), a microcolony method, an ATP (luciferase) method, a fluorescent dye method, an autofluorescence method, and so on. Among these detecting methods, a detection method enabling to obtain the result on the presence/absence of the viable particles in real time is the autofluorescence method. This autofluorescence method is a detection method of viable particles utilizing an autofluorescence emitted from the viable particles. A concrete autofluorescence method detects the viable particles by utilizing the following phenomenon. This phenomenon is a phenomenon that, when a light with a predetermined wavelength is first radiated to a given substance, an energy state that this substance has is excited (the substance absorbs the radiated light), and thereafter the substance emits extra energy to the outside as fluorescence at the time of returning to a ground state from the excited state. Further, when irradiated with a light having a wavelength unique to the substance, the substance easily emits an autofluorescence to the outside. This is because the wavelength easily causing the substance to absorb the light differs depending on each substance.

There has been known a prior art for confirming the presence/absence of viable particles in water by utilizing this autofluorescence phenomenon (refer to Japanese translation of PCT International Application No. 2009-501907: Patent Document 1). In this prior art, the presence/absence of viable particles is confirmed based on whether or not an autofluorescence is detected. For this purpose, in this prior art, an ultraviolet ray is radiated to a water. In particular, this prior art uses a filter that selects a specific part (wavelength band) of the autofluorescence that is to be measured.
Patent Document 2 describes an apparatus and method for detection of fluorescent particles in a fluid. The fluid containing particles is illuminated by a laser beam and bursts of photons from the particles are detected using a spectral filter and a photomultiplier tube. The spectral filter is used to reject background scattered Rayleigh and Raman emission.
Patent Document 3 describes an apparatus for extracorporeal blood treatment. This apparatus is disposed within a discharge unit connected to a dialyzer and estimates an absorption of substances excreted by urine in the spent dialysis fluid using a radiation source and a detector. Thereby, the spent dialysis fluid is monitored during the treatment.
Patent Document 4 describes a system for monitoring water quality for a dialysis fluid. In this system, several MEMS sensors are placed in the fluid line, one of which is placed upstream of the dialyzer. These MEMS sensors are used to sense and measure levels of contaminants or other species within the dialysis fluid entering the patient.[Prior Art]

### [Patent Document]

[Patent Document 1] Japanese translation of PCT International Application No. 2009-501907
[Patent Document 2] WO 90/14589 A1
[Patent Document 3] EP 2 218 472 A1
[Patent Document 4] WO 2010/024963 A1
[Patent Document 5] Japanese Patent Application Laid-open No. 2007-300840
[Patent Document 6] Japanese Patent Application Laid-open No. 2009-673

### [Disclosure of the Invention]

### [Problems to be Solved by the Invention]

In the detection of the viable particles utilizing the auto fluorescence phenomenon in water as in the art of Patent Document 1, a Raman-scattered light by the water is also detected in addition to the autofluorescence. This is because the radiated ultraviolet ray is scattered by the water (Raman scattering) when the ultraviolet ray is radiated to the water. Concretely, the Raman-scattered light longer in wavelength than the radiated ultraviolet ray is emitted. Therefore, it becomes difficult to detect the presence/absence of only the viable particles by using the autofluorescence as an index. Further, even if the autofluorescence only whose specific region is selected is detected, it is difficult to detect the presence/absence of the viable particles. This is because the Raman-scattered light which has the same wavelength as that of the autofluorescence is emitted from the liquid, depending on the wavelength of the radiated ultraviolet ray.

Therefore, the present invention provides an art that reduces an influence of a Raman-scattered light caused by a dialysis fluid, thereby capable of detecting viable particles in the dialysis fluid to count the number of the viable particles.

### [Solution to Problems]

In order to solve the aforesaid problem, the present invention provides a viable particle counter with the features of claim 1 (solution 1) and a dialysis fluid monitoring system with the features of claim 5.

For example, the counting of the viable particles by the viable particle counter (77) of the present invention has the following features and progresses according to a predetermined procedure.
(1) Assuming that a liquid containing a target is flowing in a flow cell, the liquid containing the target flowing in the flow cell is irradiated with a light with a specific wavelength. For example, a laser light with a single wavelength is radiated from a laser diode. Here, the target represents a viable particle, a non-viable particle, or the like. Further, the liquid represents, for example, water.
(2) The irradiation with the laser light in the above (1) results in the emission of lights due to an interaction of the laser light and the target or an interaction of the laser light and the liquid. Concretely, main emitted lights are a scattered light emitted due to reflection or the like by the viable particle, an autofluorescence emitted by utilizing energy generated by the absorption of the laser light by the viable particle, a scattered light emitted due to reflection or the like by the non-viable particle, and a Raman-scattered light emitted as a result that the wavelength of the light incident on the liquid (molecule) is converted. Note that the wavelength of the radiated laser light is set so that a peak wavelength in a spectrum of the autofluorescence becomes different from peak wavelengths of the scattered lights and the Raman-scattered light.
(3) The wavelengths of the lights emitted in the above (2) are different from one another, and for example, the scattered light from the viable particle or the non-viable particle is about equal in wavelength to the laser light, while the lights such as the Raman-scattered light and the autofluorescence are longer in wavelength than the laser light. Further, as for the wavelengths of the Raman-scattered light and the autofluorescence, their wavelength distribution regions sometimes overlap with each other, and therefore, for example, a peak value (peak wavelength) of the wavelength distribution of the autofluorescence is sometimes longer than a peak value (peak wavelength) of the wavelength distribution of the Raman-scattered light. By utilizing the fact that the peak wavelengths are made different between the autofluorescence and the Raman-scattered light by the setting of the wavelength of the radiated laser light, it is possible to make an amount of the Raman-scattered light smaller than an amount of the autofluorescence by using an optical separator using a specific wavelength (cutoff wavelength) as a reference. For example, by using a long-pass filter using a specific wavelength as a reference or a band-pass filter, the Raman-scattered light is reduced and most of the autofluorescence is transmitted.
(4) Based on the light transmitted in the above (3), that is, the autofluorescence from the viable particle, it is determined whether or not the target contained in the liquid is the viable particle. Then, when it is determined in this determination that the target is the viable particle, the number thereof is counted, so that a viable particle number is counted.
   As described above, it is possible to determine whether or not the viable particle exists in the liquid while reducing an influence of the Raman-scattered light caused by the liquid. This is because, by using the irradiating light that causes the Raman-scattered light by the liquid and the autofluorescence to be different in peak wavelength, it is possible to optically reduce the Raman-scattered light and on the other hand to transmit the autofluorescence from the viable particle. Consequently, it is possible to improve counting accuracy of the viable particles. The following solutions shall be interpreted only as examples that are not comprised by the present invention.
   Solution 2: The viable particle counter (77) of the present invention is the viable particle counter (77) according to the solution 1, further including a scattered light selecting optical means (60) which reflects a scattered light emitted from the target and transmits the light including the autofluorescence and the Raman-scattered light, wherein the viable particle determining means (2) determines whether or not the target contained in the liquid is the viable particle, based on the light transmitted by both the scattered light selecting optical means (60) and the autofluorescence selecting optical means (70).
   For example, the counting of the viable particles by the viable particle counter (77) of this solution has the following features and progresses according to a predetermined procedure.
(5) According to the above (3), as for the wavelengths of the lights emitted in the above (2), the wavelengths of the lights such as the Raman-scattered light and the autofluorescence are longer than the wavelength of the radiated laser light. Note that the wavelength of the scattered light from the viable particle or the non-viable particle is about equal to the wavelength of the laser light. Therefore, the wavelength of the scattered light from the viable particle or the non-viable particle is shorter than those of the Raman-scattered light and the autofluorescence. By utilizing this wavelength relation, it is possible to select the scattered light from the viable particle or the non-viable particle by using an optical separator using a specific wavelength as a reference. For example, by using a short-pass filter using a specific wavelength (cutoff wavelength) as a reference, it is possible to transmit only the scattered light from the viable particle or the non-viable particle. Alternatively, by using a dichroic mirror, the scattered light from the viable particle or the non-viable particle can be separated from the Raman-scattered light and the autofluorescence based on the cutoff wavelength. Concretely, by using a specific cutoff wavelength that the dichroic mirror has as a reference, the lights are separated into two by the reflection and the transmission. For example, the scattered light from the viable particle or the non-viable particle is reflected by the dichroic mirror because it has a wavelength shorter than the cutoff wavelength of the dichroic mirror. On the other hand, the Raman-scattered light and the autofluorescence are transmitted by the dichroic mirror because they have wavelengths longer than the cutoff wavelength of the dichroic mirror.
(6) Based on the mutual relation between the light separated in the above (3) (autofluorescence) and the light separated in the above (5) (the scattered light from the viable particle or the non-viable particle), it is determined whether or not the target contained in the liquid is the viable particle. Then, when the target is determined as the viable particle in this determination, the number thereof is counted, so that the viable particle number is counted. Further, when the target is determined as the non-variable particle, the number thereof may also be counted.
(7) Here, the long-pass filter in the above (3) may be installed on a downstream transmission side of the dichroic mirror in the above (5). As for the separation of the lights by the above (3) and (5), the separation by the above (5) may be performed first, and then for the light obtained after the separation, the separation by the above (3) may be performed. Concretely, first, the scattered light from the viable particle or the non-viable particle whose wavelength is shorter than the cutoff wavelength is reflected and the light including the Raman-scattered light and the autofluorescence whose wavelengths are longer than the cutoff wavelength is transmitted. This indicates the separation of the reflected light and the transmitted light in the above (5). Next, out of the transmitted light including the Raman-scattered light and the autofluorescence, only the autofluorescence whose wavelength is longer than the cutoff wavelength is transmitted. This indicates the separation of the transmitted light and the non-transmitted light in the above (3).
(8) Based on the mutual relation between the light finally separated (transmitted) by the above (7) (autofluorescence) and the light separated (reflected) on the middle stage (the scattered light from the viable particle or the non-viable particle), it is determined whether or not the target contained in the liquid is the viable particle. Then, when the target is determined as the viable particle in this determination, the number thereof is counted, so that the viable particle number is counted, and when the target is determined as the non-viable particle, the number thereof may also be counted.
   As described above, according to this solution, it is possible to determine whether or not the target contained in the liquid is the viable particle based on the mutual relation with the scattered light from the target. This is because the transmission of the Raman-scattered light is reduced and on the other hand, the autofluorescence from the viable particle is transmitted. For example, when the scattered light and the transmitted light exist, it can be determined that the target is the viable particle. Further, when only the scattered light exists, it can be determined that the target is the non-viable particle but not the viable particle. Further, when the transmitted light exists, it can be determined that this light is the Raman-scattered light caused by water. Consequently, it is possible to further improve counting accuracy of the viable particles. Further, according to an installation environment (condition), it is possible to use the optical separator such as the dichroic mirror, the long-pass filter, the band-pass filter, the short-pass filter, or the like.
   Solution 3: The viable particle counter (77) of the present invention is the viable particle counter (77) according to the solution 2, further including: an autofluorescence receiving means (90) which receives the light transmitted by the autofluorescence selecting optical means (70) to output a first signal having an intensity corresponding to an amount of the received light; a scattered light receiving means (110) which receives the light having undergone the scattered light selecting optical means (60) to output a second signal having an intensity corresponding to an amount of the received light; a scattered light detection signal outputting means (300) which, when the intensity of the second signal output by the scattered light receiving means (110) is equal to or larger than a predetermined threshold value, determines that the scattered light emitted from the target contained in the liquid is detected and outputs a detection signal; and a light shielding wall (65) which prevents a light incident from a place other than a optical path from entering the optical path, which leads from the scattered light selecting optical means (60) to the autofluorescence receiving means (90) through the autofluorescence selecting optical means (70), wherein, in the case where the detection signal is output by the scattered light detection signal outputting means (300), when the light is received by the autofluorescence receiving means (90) at the same time as an instant when the scattered light emitted from the target contained in the liquid is received by the scattered light receiving means (110) and the intensity of the first signal corresponding to the light received by the autofluorescence receiving means (90) at the same time as the instant is equal to or larger than a predetermined threshold value, the viable particle determining means (2) determines that the target contained in the liquid corresponding to the detection signal output by the scattered light detection signal outputting means (300) is the viable particle.
   For example, the counting of the viable particles by the viable particle counter (77) of this solution has the following features and progresses according to a predetermined procedure.
(9) The light transmitted in the above (3) (autofluorescence) is received and the first signal having the intensity corresponding to the amount of the received light is output. For example, the first signal corresponding to the amount of the received light is output by a light detecting device such as a photo multiplier tube or a photodiode.
(10) The light reflected in the above (5) (scattered light by the target) or the light reflected in the above (7) (the scattered light by the target) is received and the second signal having the intensity corresponding to the amount of the received light is output. For example, the second signal corresponding to the amount of the received light is output by a light detecting device such as a photodiode.
(11) It is determined whether or not the intensity of the second signal output in the above (10) is equal to or larger than the predetermined threshold value, and when as a result of the determination it is determined that the intensity is equal to or larger than the threshold value, the detection signal indicating that the scattered light from the target is detected (for example, a detection flag) is output.
(12) It is confirmed whether or not the first signal is output as a result that the light separated in the above (3) or transmitted in the above (8) is received at the time corresponding to the detection signal, that is, at the same time as an instant when the light is received in the above (10), in the case where the detection signal is output in the above (11). Then, when the first signal is output, it is determined in the determination in the above (4) that the target is the viable particle. Incidentally, when the detection signal is output and the first signal is not output, it may be determined that the target is the non-viable particle. Then, when it is determined in this determination that the target is the viable particle, the number thereof is counted, so that the viable particle number is counted. Incidentally, when it is determined that the target is the non-viable particle, the number thereof may be also counted. Further, the size of the viable particle (or the non-viable particle) may be determined based on the intensity of the second signal.
   As described above, according to this solution, by providing the predetermined threshold value for the scattered light by the target, it is possible to determine that the signal having a larger intensity than the threshold value indicates the scattered light.
   Solution 4: The viable particle counter (77) of the present invention is the viable particle counter (77) according to any one of the solution 1 to the solution 3, wherein the predetermined wavelength of the light radiated from the light emitting means (10) is 375 nm to 420 nm (when the liquid is water), and a cutoff wavelength serving as a reference for the light transmission by the autofluorescence selecting optical means (70) is 450 nm to 490 nm.
   For example, the counting of the viable particles by the viable particle counter (77) of this solution has the following features and progresses according to a predetermined procedure.
(13) The wavelength of the laser light radiated by the above (1) is set to 375 nm to 420 nm, and the cutoff wavelength for reducing the Raman-scattered light by the water and for transmitting the autofluorescence by the above (3) or the above (8) is set to a predetermined wavelength between 450 nm to 490 nm.
   As described above, according to this solution, it is possible to further improve the counting accuracy of the viable particles. This is made possible by using the autofluorescence from riboflavin in a cell of the viable particle in the water as an index. For this purpose, the 405 nm laser light is radiated in order to easily excite an energy state of the riboflavin. Thereafter, the autofluorescence whose peak wavelength is about 520 nm is emitted from the riboflavin, and the Raman-scattered light whose peak wavelength is about 465 nm is emitted from the water. Therefore, by setting the cutoff wavelength serving as a reference of the autofluorescence selecting optical means (70) (long-pass filter) to 490 nm, it is possible to efficiently separate the autofluorescence and the Raman-scattered light.
   Solution 5: The viable particle counter (77) of the present invention is the viable particle counter (77) according to any one of the solution 1 to the solution 4, further including: a liquid distributing means (720) which distributes a dialysis fluid for blood dialysis or blood filtration; and a liquid dividing means (725) which makes part of the dialysis fluid distributed by the liquid distributing means (720) branch off as the liquid containing the detection target before the dialysis fluid is administered to a human body, wherein the light emitting means (10) radiates a light with a predetermined wavelength toward the dialysis fluid made to branch off by the liquid dividing means (725), and based on the autofluorescence out of the lights emitted due to an interaction between the target contained in the dialysis fluid and the light radiated from the light emitting means (10), the viable particle determining means (2) determines whether or not the target contained in the dialysis fluid is the viable particle.
   For example, the counting of the viable particles by the viable particle counter (77) used in the inspection of the dialysis fluid of the present invention has the following features and progresses according to a predetermined procedure.
(14) A dialysis fluid before it is administered to a human body (before it is used for blood dialysis) is partly collected. The purpose of this is to inspect whether or not a viable particle exists in the dialysis fluid. Concretely, a dialysis fluid before it flows into a blood purifying device which artificially purifies blood, for example, a dialyzer performing blood dialysis or blood filtration dialysis, or a dialysis fluid before it is mixed into blood having undergone blood filtration corresponds to the aforesaid dialysis fluid. Then, the collected dialysis fluid is irradiated with a light with a predetermined wavelength. For example, a laser light with a single wavelength is radiated from a laser diode. Here, a dialysis fluid necessary for blood dialysis or blood filtration contains components deficient in a human body, but since it is mostly made of water, it is assumed in the description below that the dialysis fluid is the water. Further, a target being a detection target contained in the dialysis fluid (water) is a viable particle, a non-viable particle, or the like.
(15) When the laser light is radiated by the above (14), lights are emitted due to an interaction of the laser light and the target. Incidentally, main emitted lights are concretely a scattered light emitted due to the reflection or the like by the viable particle, an autofluorescence emitted by utilizing energy caused by the absorption of the laser light by the viable particle, a scattered light emitted due to the reflection or the like by the non-viable particle.
(16) Based on the autofluorescence from the viable particle by the above (15), it is determined whether or not the target contained in the water is the viable particle. Then, when it is determined in this determination that the target is the viable particle, the number thereof is counted, so that the viable particle number is counted.
   As described above, according to this solution, it is possible to determine in real time whether or not the viable particle exists. This is because regarding the dialysis fluid not having undergone the blood dialysis or the dialysis fluid not having been administered to the human body, the autofluorescence from an autofluorescent substance serving as an index is detected. Further, since it can be confirmed in real time at a site where the blood dialysis is executed that the viable particle exists in the dialysis fluid, it is possible to take a quick measure to the dialysis fluid before it gets into an abnormal state (a large number of the viable particles are counted), which can reduce an influence on a patient to the minimum.
   Solution 6: A dialysis fluid monitoring system of the present invention is a dialysis fluid monitoring system including the viable particle counter (77) according to the solution 5, the system including: a dialysis fluid supplying means (630) for many people which supplies the dialysis fluid for a plurality of people; a liquid distributing means (720m) for many people which divides and distributes the dialysis fluid supplied by the dialysis fluid supplying means (630) for many people to monitoring devices (710); and a notifying means (76, 400, 640, 710) which notifies a determination result by the viable particle counter (77), wherein the viable particle counter (77) is provided in at least one of the dialysis fluid supplying means (630) for many people and the plural dialysis monitoring devices (710) and, regarding the dialysis fluid flowing in the dialysis fluid supplying means (630) for many people or the dialysis fluid divided to the dialysis monitoring devices (710), determines whether or not the target contained in the dialysis fluid is a viable particle.
   For example, the inspection of the viable particles in the dialysis fluid by the dialysis fluid monitoring system of the present invention has the following features and progresses according to a predetermined procedure.
(17) For example, since blood dialysis generally requires several hours, blood dialyses for a plurality of people are performed at the same time. For this purpose, the dialysis fluid supplying device for many people is provided. The dialysis fluid supplying device for many people is a device storing a large amount of the dialysis fluid in advance and is used for supplying the dialysis fluid for plural people.
(18) The dialysis fluid supplied from the dialysis fluid supplying device for many people in the above (17) is distributed through a pipe up to sites where the blood dialyses are performed. Incidentally, the pipe branches off in the middle, and the dialysis fluid flowing in the branching pipes is used for the blood dialysis, and the blood dialyses are performed at the same time for a plurality of patients. The blood dialysis is monitored by the dialysis monitoring device (710) for each of the patients, the dialysis fluid is first sent to the dialysis monitoring devices (710) from the dialysis fluid supplying device for many people, and the blood dialyses of the patients are performed through the dialysis monitoring devices (710).
(19) Here, before the blood dialysis is performed for each patient by the dialysis fluid in the above (18), part of the dialysis fluid is made to branch off and is extracted, and it is determined whether or not the viable particle exists by using each of the viable particle counters (77).
(20) The results of the determination on whether or not the viable particle exists using the viable particle counters (77) by the above (19) may be notified, for example, by the viable particle counters (77) or the dialysis monitoring devices (710) by means of the display on a screen or warning sound, or may be collected to and managed by a central monitoring and controlling device.
   As described above, according to this solution, when the blood dialyses are performed at the same time for a plurality of patients, it is possible to confirm in real time that the viable particle exists in the dialysis fluid. Further, when the viable particles or the like are confirmed in the dialysis fluid, a quick measure can be taken to the dialysis fluid, which can reduce an influence on the patients to the minimum. This is because the results that the viable particles exist are notified by the viable particle counters (77) or the dialysis monitoring devices (710) by means of the display on the screen, the warning sound, or the like.
   Solution 7: The dialysis fluid monitoring system of the present invention is the dialysis fluid monitoring system according to the solution 6, wherein the viable particle counter (77) is provided in each of the monitoring devices (710).
   For example, the inspection of the viable particle in the dialysis fluid by the dialysis fluid monitoring system of the present invention has the following feature and progresses according to a predetermined procedure.
(21) Regarding the blood dialyses performed at a plurality of sites, it is possible to confirm that the viable particle exists in the dialysis fluid in real time at these sites. This is because the viable particle counters (77) are provided in the respective dialysis monitoring devices (710) of the above (18). Therefore, a quick measure can be taken to the dialysis fluid, which can reduce the influence on the patients to the minimum.
   Solution 8: The viable particle counter (77) of the present invention is the viable particle counter (77) according to any one of the solution 1 to the solution 4, further including a dividing means (78) which makes at least one of water under purification treatment and water whose purification treatment is finished branch off as the liquid containing the target to be detected, wherein the light emitting means (10) radiates a light with a predetermined wavelength to the water made to branch off by the dividing means (78), and the viable particle determining means (2) determines whether or not the target contained in the purified water is the viable particle, based on the autofluorescence out of lights emitted due to an interaction of the target contained in the water and the light radiated by the light emitting means (10).
   For example, the counting of the viable particles by the viable particle counter (77) used in the inspection of the purified water of the present invention has the following features and progresses according to a predetermined procedure.
(22) It is inspected whether or not the viable particle exists in the water under the purification treatment or the water having undergone the purification treatment. Incidentally, the water before the purification treatment or under the purification treatment contains non-viable particles (for example, dust, sand, soil, or the like) besides the viable particles. As a method of inspecting the presence/absence of the viable particles, part of the water under the purification treatment or having undergone the purification treatment is first made to branch off. Here, the purification treatment represents, for example, chemical disinfection treatment, filtration treatment, settlement treatment, or the like. Then, the branching water is irradiated with a light with a specific wavelength. For example, a laser light with a single wavelength is radiated from a laser diode.
(23) When the laser light is radiated by the above (22), lights are emitted due to an interaction of the laser light and the target. Incidentally, main emitted lights are concretely a scattered light emitted due to the reflection or the like by the viable particle, an autofluorescence emitted by utilizing energy caused by the absorption of the laser light by the viable particle, and a scattered light emitted due to the reflection or the like by the non-variable particle.
(24) Based on the autofluorescence from the viable particle by the above (23), it is determined whether or not the target contained in the water is the viable particle. Then, when it is determined in this determination that the target is the viable particle, the number thereof is counted, so that the viable particle number is counted.
   As described above, according to this solution, it is possible to determine in real time whether or not the viable particle exists. This is because regarding the water under the purification treatment or the water having undergone the purification treatment, the autofluorescence serving as an index from an autofluorescent substance is detected. Further, when the presence of the viable particle is confirmed, it is possible to promote further purification of the water in order to eliminate the viable particle by disinfection or sterilization. This is because it is possible to confirm that the viable particle exists in the water in real time at a site where the purification treatment is executed, and therefore, it is possible to notify in real time that the purification treatment of the water is not sufficiently performed. Further, it is also possible to suggest the adjustment of an amount of chemicals necessary for the disinfection or the sterilization. This is because the adjustment can be made based on the counted number of the viable particles.
   Solution 9: The viable particle counter (77) of the present invention is the viable particle counter (77) according to the solution 8, wherein the predetermined wavelength of the light radiated by the light emitting means (10) is 375 nm to 450 nm, and the cutoff wavelength serving as the reference for the transmission of the light by the autofluorescence selecting optical means (70) is 450 nm to 600 nm.
   For example, the counting of the viable particles by the viable particle counter (77) of this solution has the following feature and progresses according to a predetermined procedure.
(25) The wavelength of the radiated laser light is set to 375 nm to 450 nm, and the cutoff wavelength for reducing the Raman-scattered light by the water and transmitting the autofluorescence is set to a predetermined wavelength between 450 nm to 600 nm.
   As described above, according to this solution, it is possible to further improve counting accuracy of the viable particles. This is made possible by using the autofluorescence from riboflavin in a cell of the viable particle in the water as an index. For this purpose, the 405 nm laser light is first radiated in order to easily excite an energy state of the riboflavin. Thereafter, the autofluorescence whose peak wavelength is about 520 nm is emitted from the riboflavin, and the Raman-scattered light whose peak wavelength is about 465 nm is emitted from the water. Therefore, by setting the cutoff wavelength serving as the reference of the autofluorescence selecting optical means (70) (long-pass filter) to 490 to 600 nm, efficient separation is possible
   Solution 10: A purified water monitoring system of the present invention is a purified water monitoring system including the viable particle counter (77) according to the solution 8 or the solution 9, the system including: a purifying means (801) which purifies water containing the target by a plurality of kinds of purification treatments in a plurality of purifying basins; a water delivering means (820) which delivers the water having undergone the plural kinds of purification treatments by the purifying means (801) to at least one distribution basin; and a notifying means (76, 400, 880) which notifies the determination result by the viable particle counter (77), wherein the viable particle counter (77) is provided in at least one place between the purifying basin where the purification treatment is first performed and the distribution basin.
   For example, the inspection of the water under the purification treatment or the water having undergone the purification treatment by the purified water monitoring system of the present invention has the following feature and progresses according to a predetermined procedure.
(26) For example, purified water is manufactured by purifying raw water taken from a water source such as a river by various kinds of purifying methods. Concretely, a plurality of kinds of the purifying methods such as disinfection, settlement, and filtration are used in a plurality of purifying basins (grit basin, raw water receiving basin, mixing basin, settling basin, filter basin). The manufactured purified water is once stored in a purified water basin. Further, it is sent to distribution basins at a plurality of places (areas) by water pumps (through water pipes) from the purified water basin.
(27) The viable particle counter (77) capable of counting the viable particles having the aforesaid features is provided in the purified water basin, the water pipe connecting the purified water basin and the purified water basin (distribution basin), or the distribution basins of the above (26).
(28) The result of the determination by the viable particle counter (77) provided in the above (27) is notified to the outside.
   As described above, according to this solution, it is possible to perform in real time not only the inspection of the water under the purification treatment or the manufactured purified water having undergone the purification treatment (for example, the purified water stored in the purified water basin) but also the inspection of the water in the distribution basins at the plural places. This is made possible by, for example, providing the plural viable particle counters (77) in the distribution basins. Further, based on the results of these inspections, it is possible to monitor the purification treatment.
   Solution 11: The purified water monitoring system of the present invention is the purified water monitoring system according to the solution 10, further including: a chemical supplying means (860) which supplies chemicals used for the purification treatment by the purifying means (801); a chemical injecting means (870) which injects the chemicals supplied by the chemical supplying means (860); chemical injection amount controlling means (872, 874, 876) which control an injection amount of the chemicals injected by the chemical injecting means (870); and a central monitoring and controlling means (880) which instructs the control by the chemical injection amount controlling means (872, 874, 876) and controls water delivery in the purification treatment, wherein the central monitoring and controlling means (880) instructs the control by the chemical injection amount controlling means (872, 874, 876) based on the determination result of the viable particle counter (77).
   For example, the inspection of the water under the purification treatment or the water having undergone the purification treatment by the purified water monitoring system of the present invention has the following features and progresses according to a predetermined procedure.
(29) In the purification in the above (26), the chemicals used for disinfecting purification (for example, sodium hypochlorite) are stored in a storage tank in advance. The stored chemicals are supplied to the purifying basin (for example, a mixing basin or the like) where the disinfecting purification is performed. A water pipe where the chemicals flow is provided with a regulating valve, which makes it possible to adjust an amount of the chemicals used in the disinfecting purification.
(30) The central monitoring and controlling device controls all the purifications in the purifying basins in the above (26) including the disinfecting purification of the above (29) and also controls the adjustment of an amount of the chemicals in the above (29).
(31) The viable particle counter (77) of the above (27) also inspects the water in the purifying basin (for example, a raw water receiving basin, a mixing basin, or the like) where the disinfecting purification by the chemicals has been performed in the above (29).
(32) When it is determined as a result of the inspection of the above (31) that the viable particle exists in the water, the determination result and a count value of the viable particles are sent to the central monitoring and controlling device. Based on the sent information, the central monitoring and controlling device performs the control for adjusting an amount of the chemicals used for the disinfecting purification of the above (29). Further, the central monitoring and controlling device also controls the delivery of the water flowing to the water pipe in the above (26).

As described above, according to this solution, by, for example, providing the plural viable particle counters (77), it is possible to also inspect the chlorinated water (for example, water stored in a raw water receiving well, or a mixing basin) in real time. Further, based on the inspection results, it is possible for the central monitoring and controlling device to decide an injection amount of chlorine in the chlorination. Consequently, it is possible to confirm an increase/decrease of seaweeds, microorganisms, and so on due to a climate change or the like by the inspection of the plural viable particle counters (77), and to adjust an injection amount of the chlorine according to the inspection results. Further, since it is possible to prevent too large an amount of the chlorine from being injected more than necessary, it is possible to reduce damage of the water pipe, and chlorination by-products (trihalomethane or the like) generated by the chlorination which affect a human body.

### [Effect of Invention]

According to the viable particle counter and the viable particle counting method of the present invention, the high-accuracy counting is possible. Further, even in a state where the Raman-scattered light by the dialysis fluid is generated, it is possible to efficiently detect the autofluorescence emitted from the viable particle in the flowing dialysis fluid.

### [Brief Description of Drawings]

[FIG. 1] FIG. 1 is a schematic block diagram illustrating one embodiment of a viable particle counter.
[FIG. 2] FIG. 2 is a flowchart illustrating a procedure example of a process of a viable particle counting method using the viable particle counter.
[FIG. 3] FIG. 3 is a chart illustrating excitation absorption spectrums of riboflavin and NAD(P)H being examples of an autofluorescent substance and spectrums of autofluorescences from the substances.
[FIG. 4] FIG. 4 is a schematic chart of a spectrum of a Raman-scattered light by water at the time of irradiation with a light with a 405 nm wavelength.
[FIG. 5] FIG. 5 is a chart illustrating an example of a time-variable intensity distribution of the Raman-scattered light by the water before it enters an optical filter.
[FIG. 6] FIG. 6 is a chart illustrating an example of a time-variable intensity distribution of the Raman-scattered light by the water after it is transmitted by the optical filter.
[FIG. 7] FIG. 7 is a chart illustrating an example of time-variable intensity distributions of all lights before they enter the optical filter.
[FIG. 8] FIG. 8 is a chart illustrating an example of time-variable intensity distributions of all the lights after they are transmitted by the optical filter.
[FIG. 9] FIG. 9 is a flowchart illustrating a procedure example of an autofluorescence counting process.
[FIG. 10] FIG. 10 is a flowchart illustrating a procedure example of a data collecting process.
[FIG. 11] FIG. 11 is a flowchart illustrating a procedure example of a data analyzing process.
[FIG. 12] FIG. 12 is a flowchart illustrating a procedure example of an analyzing process.
[FIG. 13] FIG. 13 is a chart illustrating examples of output signals from an autofluorescence receiving device and a scattered light receiving device.
[FIG. 14] FIG. 14 is a flowchart illustrating a procedure example of an analysis result outputting process.
[FIG. 15] FIG. 15 is a flowchart illustrating a procedure example of a notifying process.
[FIG. 16] FIG. 16 is a view illustrating an example of the notification of a counting result of viable particles.
[FIG. 17] FIG. 17 is an explanatory diagram of a system relating to dialysis fluid inspection in blood dialysis.
[FIG 18] FIG. 18 is an explanatory diagram of an artificial dialysis device system for many people.
[FIG. 19] FIG. 19 is an explanatory diagram of a system relating to viable particle inspection of purified water in a water purification plant.
[FIG. 20] FIG. 20 is an explanatory diagram of a purified water inspecting system using the viable particle counter.

### [Best Mode for Carrying out the Invention]

Hereinafter, embodiments of the present invention will be described with reference to the drawings.

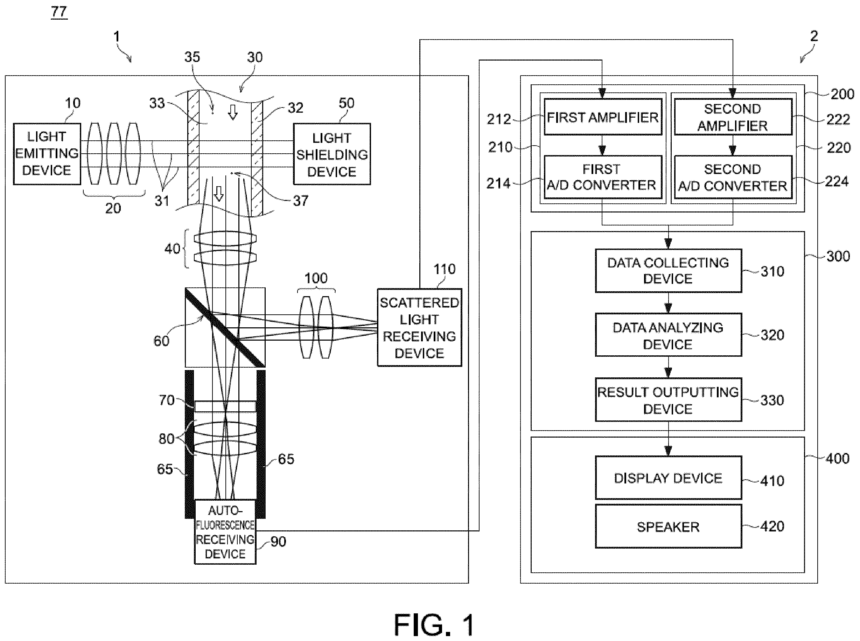

FIG. 1 is a schematic block diagram illustrating one embodiment of a viable particle counter.

As illustrated in FIG. 1, the viable particle counter 77 is composed of: a light detecting system 1 which radiates a light to a target to detect a scattered light and an autofluorescence from the target; and an autofluorescence counting system 2 which counts the number of the autofluorescences based on signals output from the light detecting system 1. By these systems, it is possible to detect and count viable particles among targets in water (hereinafter, referred to as the viable particles). Incidentally, the viable particles that can be detected (counted) in this embodiment are viable particles with, for example, a 0.1 µm to several 100 µm size, and concretely, are bacteria, yeast, mold, or the like. Further, the light radiated to the viable particles is a laser light in an ultraviolet region, and the autofluorescence emitted from a substance (riboflavin, NAD(P)H (nicotinamide adenine dinucleotide (phosphate), or the like) existing in the body (cell) of the viable particle and necessary for metabolism is detected as an index.

### [Light detecting system]

The light detecting system 1 is composed of, for example, a light emitting device 10, a radiation optical lens system 20, a target flow device 30, a first condensing optical lens system 40, a light shielding device 50, a scattered light selecting optical device 60, a light shielding wall 65, an autofluorescence selecting optical device 70, a second condensing optical lens system 80, an autofluorescence receiving device 90, a third condensing optical lens system 100, and a scattered light receiving device 110. By these constituent elements, it is possible to radiate the light to the target and detect the scattered light and the autofluorescence from the target.

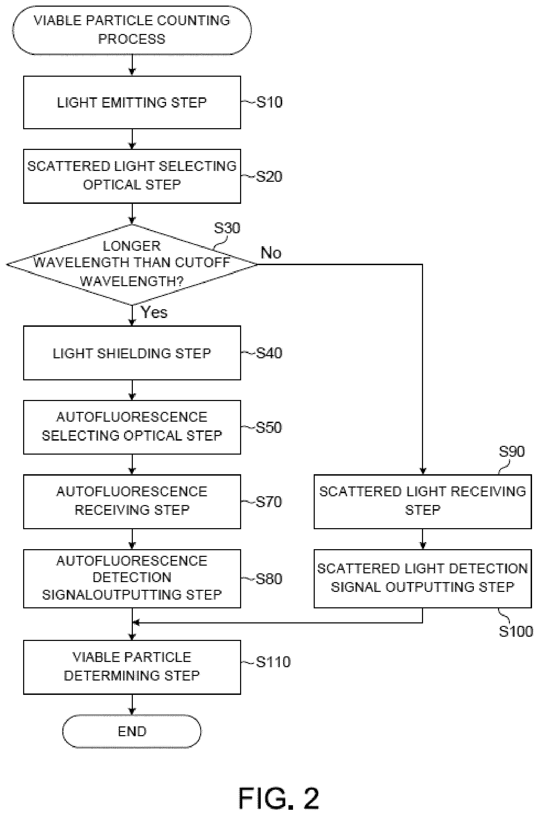

Further, FIG. 2 is a flowchart illustrating a procedure example of a process of a viable particle counting method (viable particle counting process) using the aforesaid viable particle counter 77. As illustrated in FIG. 2, in the viable particle counting process, a light emitting step (Step S10) of radiating a laser light toward a liquid as a detection target is first executed by the light emitting device 10. Next, a scattered light selecting optical step (Step S20) of separating lights is executed by the scattered light selecting optical device 60. The light separation of this scattered light selecting optical step (Step S20) is performed based on whether or not lights obtained after the light emitting step (Step S10) (the autofluorescence and the scattered light from the viable particle and a Raman-scattered light of the liquid which are caused by the laser light) have a wavelength longer than a predetermined cutoff wavelength. Next, a subsequent process is separated depending on whether or not the light having undergone the scattered light selecting optical step (Step S20) has a wavelength longer than the cutoff wavelength (whether or not it is transmitted or reflected by the scattered light selecting optical device 60) (Step S30). Here, a light shielding step (Step S40) of preventing an external light from entering is executed by the light shielding wall 65. This light shielding step is performed for the transmitted light (Step S30: Yes) having undergone the scattered light selecting optical step (Step S20) inside a optical path up to the autofluorescence receiving device 90 (Step S70). Next, regarding the transmitted light, an autofluorescence selecting optical step (Step S50) of selecting a light having a longer wavelength than a predetermined cutoff wavelength is executed by the autofluorescence selecting optical device 70. Then, an autofluorescence receiving step (Step S70) of receiving the light obtained after the autofluorescence selecting optical step (Step S50) is executed by the autofluorescence receiving device 90. Further, an autofluorescence detection signal outputting step (Step S80) of outputting a signal based on the received light is executed by the autofluorescence receiving device 90. On the other hand, regarding the reflected light (Step S30: No), a scattered light receiving step (Step S90) of receiving the reflected light is executed by the scattered light receiving device 110. Further, a scattered light detection signal outputting step (Step S100) of outputting a signal based on the received light is executed by the scattered light receiving device 110. Then, a viable particle determining step (Step S110) using the signals output in the autofluorescence detection signal outputting step (Step S80) and the scattered light detection signal outputting step (Step S100) is executed by the autofluorescence counting system 2. Further, finally, the detection and counting of the viable particles are executed by this viable particle determining step (Step S110). By the viable particle counting method composed of a series of these processes, it is possible to detect and count the viable particles in the liquid.

Hereinafter, the constituent elements included in the viable particle counter 77 and the procedures of the viable particle counting method will be concretely described.

### [Light emitting device (light emitting means, light emitting step (S10))]

The light emitting device 10 is formed by, for exmaple, a semiconductor laser diode (including a semiconductor LED element. Hereinafter, referred to as a laser diode). By the laser diode 10, the laser light is oscillated and radiated to the liquid containing the viable particles. A wavelength of the laser light oscillated by the laser diode 10 is decided according to a substance existing in the cell of the viable particle and capable of emitting the autofluorescence (hereinafter, referred to as an autofluorescent substance). Here, the autofluorescent substance has an excitation wavelength with which energy of the radiated light is easily absorbed to facilitate the excitation to the excited state. The excitation wavelength differs depending on the substance, and further, a wavelength of the autofluorescence emitted at the time of the return to a ground state from the excited state also differs depending on the autofluorescent substance. The excitation wavelength of the autofluorescent substance and the autofluorescence wavelength will be described, taking concrete examples.

### [Excitation wavelength and autofluorescence wavelength]

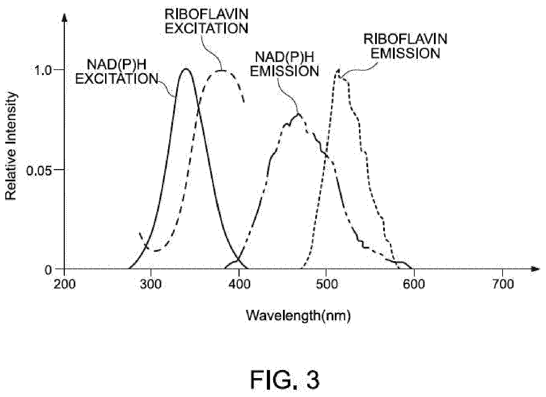

FIG. 3 is a chart illustrating examples of excitation absorption spectrums of the autofluorescent substances and spectrums of the autofluorescences from the substances.

As illustrated in FIG. 3, these distributions represent an excitation wavelength spectrum of NAD(P)H, an excitation wavelength spectrum of riboflavin, a spectrum of an autofluorescence from NAD(P)H, and a spectrum of an autofluorescence from riboflavin. For example, the excitation wavelength spectrum of NAD(P)H presents a distribution having a peak at an about 340 nm wavelength. Further, the excitation wavelength spectrum of riboflavin presents a distribution having a peak at an about 375 nm wavelength. This indicates that the radiation of a laser light having a 375 nm to 420 nm wavelength is suitable for facilitating the excitation of riboflavin.

Thus, the wavelength of the laser light oscillated from the laser diode 10 is decided according to the excitation wavelength of NA(P)H or riboflavin existing in the cells of the viable particles. Consequently, it is possible to cause the emission of many autofluorescences from the viable particles. In this embodiment, it is assumed that the laser light having a 405 nm wavelength is oscillated from the laser diode 10. The radiation of this laser light having the 405 nm wavelength causes the emission of the autofluorescences by the riboflavin from the viable particles.

### [Radiation optical lens system]

The radiation optical lens system 20 is composed of, for exmaple, a plurality of kinds of optical lenses. For example, it is composed of a collimator lens, a biconvex lens, and a cylindrical lens, and adjusts the laser light oscillated from the laser diode 10 to parallel rays to radiate them to the target.

### [Target flow device]

The target flow device 30 (flow cell 30) is formed by, for example, a tube part 32 which is a hollow quadratic prism made of synthetic quartz, sapphire, or the like, and has a structure in which a liquid 33 containing the target (viable particles 35 or non-viable particles 37) flows from top to bottom. The laser light 31 oscillated from the laser diode 10 is radiated to a hollow area of the tube part 32 in which the liquid flows, so that a detection area is formed.

In this detection area, the laser light 31 interacts with water (water molecules) of the liquid 33 flowing in the flow cell 30 and with the target (the viable particle 35 or the non-viable particle 37).

The scattered light from the viable particle 35 is also emitted with a 405 nm wavelength. This is because the wavelength of the laser light 31 incident on the viable particle 35 is 405 nm. Further, as illustrated in FIG. 3, when the laser light 31 is absorbed by riboflavin in the cell of the viable particle 35, the wavelength distribution has a peak at about 520 nm. Here, the scattered light or the autofluorescence emitted from the viable particle 35 is emitted to a surrounding area.

Further, the scattered light by the laser light 31 incident on the non-viable particle 37 is also similar to the scattered light emitted from the viable particle 35.

As described above, the scattered light from the viable particle 35 or the non-viable particle 37, or the autofluorescence from the viable particle 35 is emitted. This is because the viable particle 35 or the non-viable particle 37 interacts with the laser light 31. Then, these lights are detected by the light receiving devices. Further, before reaching the light receiving devices, these lights pass through the plural condensing lens systems and the wavelength selecting optical devices. Incidentally, the intensity of the scattered light, that is, a light amount of the scattered light depends on the size of the viable particle 35 or the non-viable particle 37, and the larger the size, the larger the light amount. Here, the autofluorescence from the viable particle 35 depends on an amount of the riboflavin in the cell of the viable particle 35. It also depends on a light amount (intensity) of the laser light 31, and by increasing laser power and radiating the laser light 31 in large quantity to the flow cell 30, the scattered light from the viable particle 35 or the non-viable particle 37 and the autofluorescence from the viable particle 35 also increase. However, also increases, concretely, a light (Raman-scattered light) due to the interaction between the laser light 31 and the water 33 (Raman scattering) also increases. Next, the Raman-scattered light by the water will be concretely described.

### [Raman scattering by water]

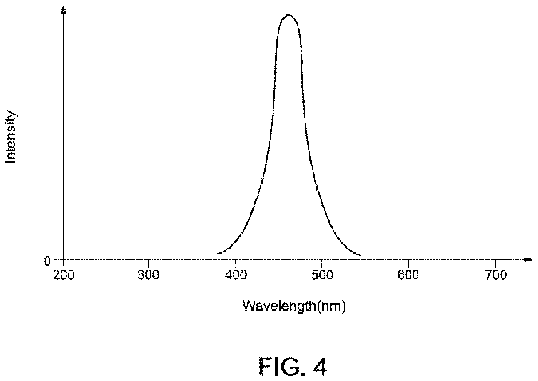

FIG. 4 is a chart illustrating an example of a spectrum of the Raman-scattered light by the water when the light having the 405 nm wavelength is radiated. As illustrated in FIG. 4, when the laser light 31 having the 405 nm wavelength is radiated to the water, the Raman-scattered light whose wavelength distribution has a peak at an about 465 nm wavelength is emitted due to the interaction between the water and the laser light 31.

### [Light shielding device]

The light shielding device 50 is formed by, for exmaple, a laser trap. For example, even when the laser light 31 is oscillated from the laser diode 10, part thereof passes without undergoing the interaction in the flow cell 30. This laser light 31 which has passed is shut off by the laser trap 50. By this light shielding, the laser light 31 which has passed is prevented from being reflected at various places, and is inhibited from becoming noise in the detection of the scattered light and the autofluorescence of the viable particles 35.

### [First condensing optical lens system]

The first condensing optical lens system 40 is composed of, for exmaple, a plurality of optical lenses. The first condensing optical lens system 40 is installed at an about 90 degree position relative to a travelling direction (optical axis) of the laser light 31. By the first condensing optical lens system 40, the scattered light from the viable particle 35 or the non-viable particle 3 in the flow cell 30 and the autofluorescence from the viable particle 35 are gathered. Incidentally, in order to gather as many side scattered lights and autofluorescences as possible from the viable particles 35, a lens aperture is preferably larger, and is decided according to a position (distance) at which a detecting device which detects the scattered light and the autofluorescence from the viable particle 35 is provided.

### [Scattered light selecting optical device (scattered light selecting optical means, scattered light selecting optical step (S20))]

The scattered light selecting optical device 60 is formed by, for example, a dichroic mirror. The dichroic mirror 60 of this embodiment transmits a light having a wavelength longer than 410 nm and reflects a light having a wavelength shorter than 410 nm. A specific wavelength as a reference for such light separation based on the wavelength will be called a cutoff wavelength. Here, the wavelength of the scattered light from the viable particle 35 or the non-viable particle 37 scattered by the 405 nm laser light 31 in the flow cell 30 is mainly 405 nm. Therefore, the dichroic mirror 60 is capable of reflecting the scattered light from the viable particle 35 or the non-viable particle 37. Further, the reflected scattered light from the viable particle 35 or the non-viable particle 37 form an image on the scattered light receiving device 110. Incidentally, this image formation is caused by the third condensing optical lens system 100 gathering the lights.

On the other hand, regarding the autofluorescence emitted from the viable particle 35 flowing in the flow cell 30, its wavelength distribution has a peak at about 520 nm as illustrated in FIG. 3. Therefore, the autofluorescence is mostly transmitted without being reflected by the dichroic mirror 60. Similarly, the wavelength distribution of the Raman-scattered light by the water has a peak at about 465 nm as illustrated in FIG. 4. Incidentally, most of the Raman-scattered light except part thereof is transmitted by the dichroic mirror 60. This is because most of the Raman-scattered light has a wavelength longer than the 410 nm cutoff wavelength of the dichroic mirror 60. Then, the transmitted autofluorescence and Raman-scattered light by the water next progress to the autofluorescence selecting optical device.

Note that the cutoff wavelength serving as the reference of the dichroic mirror 60 is not limited to 410 nm and may be such a wavelength that the scattered light scattered from the viable particle 35 or the non-viable particle 37 by the laser light 31 is reflected and the autofluorescence from the viable particle 35 is transmitted.

### [Autofluorescence selecting optical device (autofluorescence selecting optical means, autofluorescence selecting optical step (S50))]

The autofluorescence selecting optical device 70 is formed by, for example, an optical filter. In this embodiment, a long-pass filter 70 which transmits a light having a wavelength longer than a 490 nm wavelength (cutoff wavelength) is provided.

On the other hand, about 90% of the Raman-scattered light by the water can be cut off by the long-pass filter 70. This is because most of the Raman-scattered light except part thereof has a wavelength shorter than the 490 nm cutoff wavelength as illustrated in FIG. 4.

### [Change of intensity distribution of Raman-scattered light of water by long-pass filter]

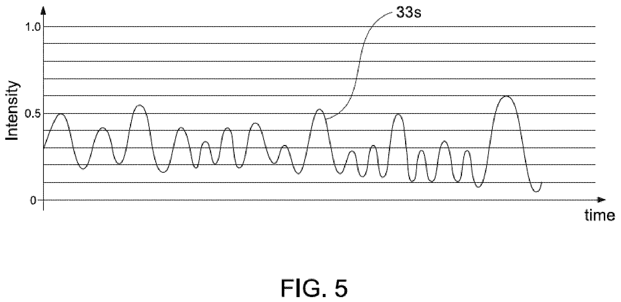

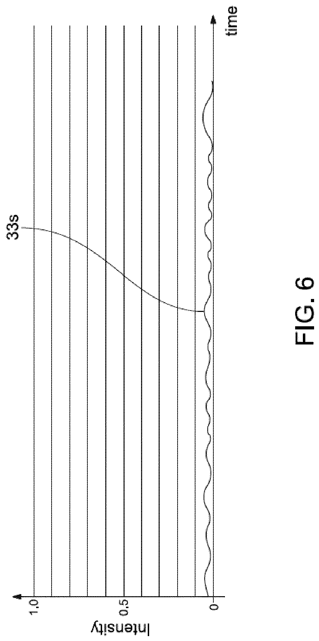

FIG. 5 is a chart illustrating an example of a time-variable intensity distribution of the Raman-scattered light by water before it enters the long-pass filter, and FIG. 6 is a chart illustrating an example of a time-variable intensity distribution of the Raman-scattered light by the water after it is transmitted by the long-pass filter. FIG. 5 and FIG. 6 show that the intensity distribution of the Raman-scattered light 33s by the water is changed by the long-pass filter.

### [Intensity distribution of Raman-scattered light by water before it enters long-pass filter]

In FIG. 5, the horizontal axis represents time and the vertical axis represents intensity of the light expressed in an arbitrary unit. It is shown that the Raman-scattered light 33s by the water presents a distribution where a random increase/decrease is repeated. For example, it is shown that the Raman-scattered light 33s by the water sometimes presents a distribution where a peak of its intensity is 0.5 and sometimes presents a distribution where the peak is 0.3, and an amount of the entering Raman-scattered light 33s by the water is sometimes large and in some other times, is small. This indicates that the Raman-scattered light 33s enters at random irrespective of the time.

### [Distribution of Raman-scattered light by water after it is transmitted by long-pass filter]

Further, in FIG. 6, the horizontal axis represents time and the vertical axis represents intensity of the light expressed in an arbitrary unit. It is shown that the intensity of the Raman-scattered light 33s by the water after it is transmitted by the long-pass filter 70 changes to about 1/10 of that in the time-variable intensity distribution of the Raman-scattered light 33s by the water illustrated in FIG. 5.

Next, not only the Raman-scattered light 33s by the water but also a light Lt transmitted by the dichroic mirror will be described. The light Lt includes the Raman-scattered light 33s by the water and the autofluorescence 35e emitted from the viable particle 35.

### [Change of intensity distribution of light by long-pass filter]

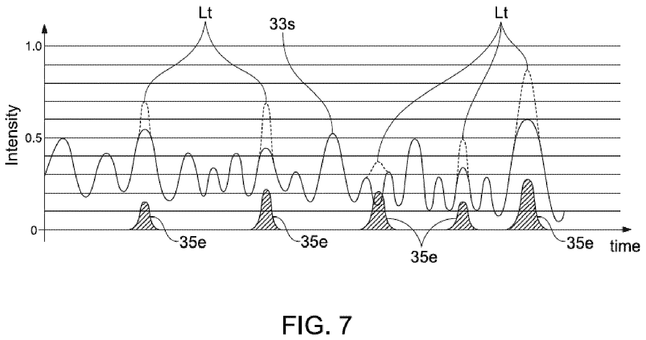

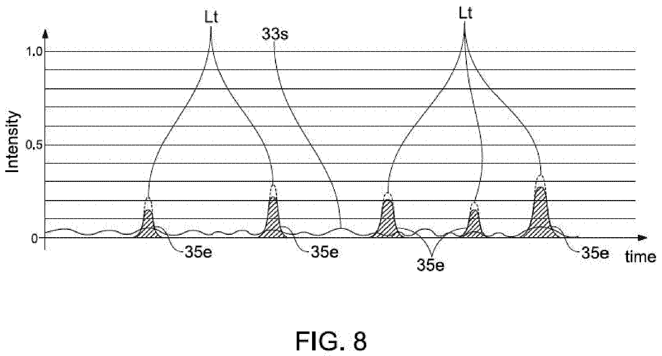

FIG. 7 is a chart illustrating an example of time-variable distributions of all the lights before they enter the optical filter, and FIG. 8 is a chart illustrating an example of time-variable distributions of all the lights after they are transmitted by the optical filter. In FIG. 7 and FIG. 8, it is seen that an intensity distribution of the total light Lt transmitted by the dichroic mirror 60 is separated by the long-pass filter 70.

### [Intensity distribution of light before it enters long-pass filter]

In FIG. 7, the horizontal axis represents time and the vertical axis represents relative intensity of the light. The distribution of the light entering the long-pass filter 70 is the distribution of the light Lt. Concretely, the light Lt is the combination (sum) of the distribution of the Raman-scattered light 33s by the water and the distribution of the autofluorescence 35e. For example, it is assumed that the Raman-scattered light 33s by the water in whose light amount distribution a relative intensity peak is 0.5 and the autofluorescence 35e in whose light amount distribution a relative intensity peak is 0.2 enter during a time period Δt from a given time instant. In this case, the light Lt whose amount is the sum of these light amounts enters during the time period Δt, and the relative intensity distribution of the light Lt is a distribution where 0.7 is a peak.

### [Intensity distribution of light after it is transmitted by long-pass filter]

Further, FIG. 8 illustrates the time-variable distribution of the total light Lt transmitted by the long-pass filter. Concretely, it illustrates a combined distribution of the time-variable intensity distribution of the light amount of the Raman-scattered light 33s by the water which becomes about 10% of that at the time of the entering and the time-variable intensity distribution of the light amount of the autofluorescence 35e which does not change from that at the time of the entering. For example, it is assumed that the Raman-scattered light 33s by the water in whose light amount distribution the peak of the relative intensity is 0.05 and the autofluorescence 35e in whose light amount distribution the peak of the relative intensity is 0.2 are transmitted during a time period Δt from a given time instant. In this case, the light Lt whose amount is the sum of these light amounts is transmitted during this time period Δt, and a relative intensity distribution of the light Lt is a distribution where the peak is 0.25.

Note that, as a wavelength reference for the light separation by the autofluorescence selecting optical device 70, a wavelength is selected so that the Raman-scattered light 33s by the water becomes less than the autofluorescence 35e emitted from the viable particle 35. Concretely, the cutoff wavelength is not limited to 490 nm and may be a wavelength having any value between 450 nm and 520 nm, preferably 450 nm and 490 nm. Further, the long-pass filter that transmits wavelengths longer than 490 nm is not restrictive, and a band-pass filter that transmits lights in a 490 nm to 600 nm wavelength band may be provided.

Here, as the autofluorescence selecting optical device 70, the long-pass filter which uses the aforesaid cutoff wavelength (for example, 490 nm) as the reference is provided. This is because the autofluorescence 35e from the riboflavin in the cell of the viable particle 35 is used as the index. On the other hand, when NAD(P)H in the cell of the viable particle 35 is used as an index, an autofluorescence selecting optical device 70 of another kind may be provided. For example, a long-pass filter whose cutoff wavelength is any wavelength (for example, 450 nm) between 410 nm and 470 nm and which transmits lights having wavelengths longer than the cutoff wavelength or a band-pass filter which transmits lights in a 450 nm to 600 nm wavelength band as a cutoff wavelength may be provided. This is because, when the laser light 31 with about 350 nm is radiated, the Raman-scattered light 33s by the water presents a distribution having a peak at 400 nm. Further, this is because, by the long-pass filter whose reference is 450 nm, it is possible to cut off most of the Raman-scattered light 33s by the water similarly to the case where the detection of the autofluorescence 35e from the riboflavin is used as an index.

### [Second condensing optical lens system: refer to FIG. 1]

The second condensing optical lens system 80 is composed of, for exmaple, a plurality of optical lenses.

The second condensing optical lens system 80 is installed on a travelling direction (optical axis) of the light transmitted by the long-pass filter 70. By this second condensing optical lens system 80, the autofluorescence 35e and the Raman-scattered light 33s by the water which are transmitted by the long-pass filter 70 are gathered, and an image is formed on a plane of incidence of the autofluorescence receiving device 90.

### [Fluorescence light receiving device (autofluorescence receiving means, autofluorescence receiving step (S70), autofluorescence detection signal outputting step (S80))]

The autofluorescence receiving device 90 is formed by, for example, a semiconductor light receiving element (photodiode: PD) or a photo multiplier tube: PMT) higher in sensitivity than the photodiode. The photodiode or the photo multiplier tube (hereinafter referred to as a photo multiplier) converts a received light to a current to output the current according to an amount of the received light. Note that intensity of the output current changes depending on the amount of the received light, and the larger the amount of the received light, the larger the intensity of the current. Note that an electric signal output from the photo multiplier 90 is next input to the autofluorescence counting system 2.

### [Light shielding wall 65 (light shielding means, light shielding step (S40))]

The light shielding wall 65 is formed by a cylindrical structure surrounding a optical path from a transmission side of the dichroic mirror 60 to the photo multiplier 90. By this light shielding wall 65, it is possible to prevent lights except the light transmitted by the dichroic mirror 60 (autofluorescence 35e) from entering the photo multiplier 90. For example, it is possible to shut off the Raman-scattered light 33s and the scattered lights 35s, 37s from the target so that these lights do not enter this optical path by being reflected in the light detecting system 1. A light shielding wall, not illustrated, may similarly be provided in a optical path from the opposite side of the dichroic mirror 60 to the scattered light receiving device 110, and so on.

### [Third condensing optical lens system]

The third condensing optical lens system 100 is composed of, for example, a plurality of optical lenses. The third condensing optical lens system 100 is installed on a travelling direction (optical axis) of the light reflected by the dichroic mirror 60.

### [Scattered light receiving device (scattered light receiving means, scattered light receiving step (S90), scattered light detection signal outputting step (S100))]

The scattered light receiving device 110 is formed by, for example, a photodiode or a photo multiplier. Here, the light entering the scattered light receiving device 110 is a light with a wavelength shorter than 410 nm reflected by the dichroic mirror 60, and concretely, the scattered light scattered by the viable particle 35 or the non-viable particle 37 flowing in the flow cell 30. The scattered light by the viable particle 35 or the non-viable particle 37 can be sufficiently detected even by an inexpensive photodiode instead of a photo multiplier. This is because the scattered light by the viable particle 35 or the non-viable particle 37 has a larger light amount than that of the autofluorescence 35e emitted from the viable particle 35. In this embodiment, the photodiode 110 is provided, which receives the scattered light by the viable particle 35 or the non-viable particle 37 reflected by the dichroic mirror 60. The light received by the photodiode 110 is converted to an electric signal according to its light amount, and the electric signal is output from the photodiode 110. The output signal from the photodiode 110 is next input to the autofluorescence counting system 2.

### [Autofluorescence counting system (viable particle determining means, viable particle determining step (S110)): refer to FIG. 1]

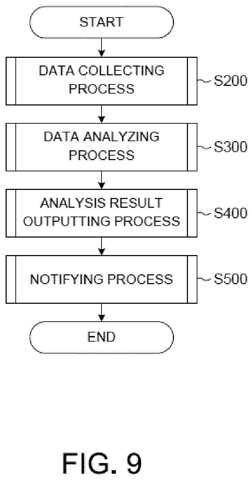

The autofluorescence counting system 2 is composed of, for example, a detection signal processing part 200, a data processing part 300, and a notifying part 400. Further, FIG. 9 is a flowchart illustrating a procedure example of an autofluorescence counting process.

The detection signal processing part 200 first receives, for example, the output signals from the light detecting system 1, that is, the output signal from the autofluorescence receiving device (photo multiplier) 90 and the output signal from the scattered light receiving device (photodiode) 110. Next, the detection signal processing part 200 amplifies the received signals. Finally, the detection signal processing part 200 performs processing of AD-converting the analog signals to digital signals, and so on (data collecting process Step S200 in FIG. 9).

For example, the data processing part 300 receives and stores an auto fluorescence signal (signal A) and a scattered light signal (signal B) obtained by the AD conversion processing by the detection signal processing part 200 (data analyzing process Step S300 in FIG. 9). Next, based on the stored signal A and signal B, the data processing part 300 determines whether or not the signal ascribable to the viable particle 35 in the liquid, that is, the signal ascribable to the autofluorescence 35e is included. Finally, the data processing part 300 performs processing of outputting the determination result (the analysis result outputting process Step S400 in FIG. 9), and so on.

For example, the notifying part 400 notifies the result determined by the data processing part 300 to the outside or outputs a notification signal to the outside (notifying process Step S500 in FIG. 9).

Hereinafter, the constituent elements and their processes will be concretely described.

### [Detection signal processing part]

For exmaple, the detection signal processing part 200 is composed of an autofluorescence-based output signal processing device 210 and a scattered light-based output signal processing device 220. Further, the autofluorescence-based output signal processing device 210 is composed of, for example, a first amplifier 212 and a first analog/digital converter 21, and the scattered light-based output signal processing device 220 is composed of, for example, a second amplifier 222 and a second analog/digital converter 224.

### [Data collecting process]

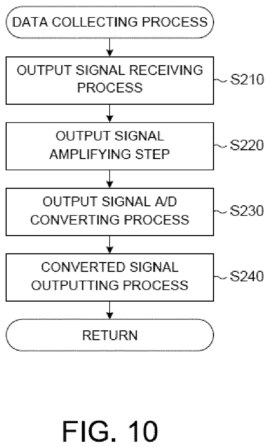

FIG. 10 is a flowchart illustrating a procedure example of the data collecting process.

First, when the autofluorescence-based output signal processing device 210 receives the output signal from the autofluorescence receiving device (photo multiplier) 90 (output signal receiving process Step S210), the first amplifier 212 amplifies the output signal output from the photo multiplier 90 (output signal amplifying process Step S220). Then, the first analog/digital converter 214 converts the analog signal amplified by the first amplifier 212 to the digital signal (signal A) (output signal A/D converting process Step S230).

Similarly, when the scattered light-based output signal processing device 220 receives the output signal from the scattered light receiving device (photodiode) 110 (output signal receiving process Step S210), the second amplifier 222 amplifies the output signal output from the photodiode 110 (output signal amplifying process Step S220). Then, the second analog/digital converter 224 converts the analog signal amplified by the second amplifier 222 to the digital signal (signal B) (output signal A/D converting process Step S230).

Thereafter, the signal A and the signal B converted to the digital signals are output from the autofluorescence-based output signal processing device 210 and the scattered light-based output signal processing device 220 (converted signal outputting process Step S240). The output signal A and signal B are next input to the data processing part 300.

### [Data processing part]

The data processing part 300 is composed of, for example, a data collecting device 310, a data analyzing device 320, and an analysis result outputting device 330. Further, the data collecting device 310 is formed by, for example, a memory (RAM) 310 which stores data.

### [Data analyzing process]

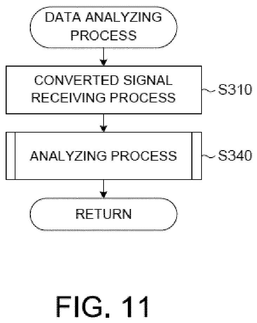

FIG. 11 is a flowchart illustrating a procedure exmaple of the data analyzing process.

First, the data processing part 300 receives the signal A and the signal B output from the autofluorescence-based output signal processing device 210 and the scattered light-based output signal processing device 220 (converted signal receiving process Step S310). The received signal A and signal B are stored in a memory area of the memory 310 as they are.

When the storage of the signal A and the signal B to the memory 310 is finished, an analyzing process (analyzing process Step S340) is next performed by using these signal A and signal B.

### [Data analyzing device (viable particle determining means, scattered light detection signal outputting means)]

The data analyzing device 320 is composed of, for example, a calculating circuit (for exmaple, CPU 322) which analyzes the data (the signal A and the signal B) stored in the memory 310 and a memory 324 (ROM) storing (saving) the calculation contents (program, threshold value data) and so on.

### [Analyzing process (viable particle determining step, scattered light detection signal outputting step)

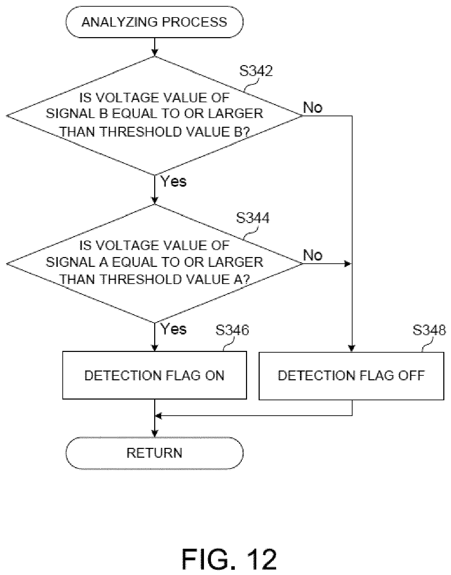

FIG. 12 is a flowchart illustrating a procedure example of the analyzing process.

First, the CPU 322 compares the signal B stored in the memory 310 with the threshold data (voltage value) stored in advance in the memory 324. Concretely, it is determined whether or not a voltage value of the stored signal B is equal to or larger than a threshold value B (VthB) (Step S342). When, as a result of this determination, it is determined that the voltage value of the signal B is equal to or larger than the threshold value B (Step S342: Yes), this indicates that the scattered light from the viable particle 35 or the non-viable particle 37 has entered the scattered light receiving device photodiode 110 and is detected. Here, a process of setting a scattered light detection flag indicating that the scattered light from the viable particle 35 or the non-viable particle 37 is detected to ON may be performed.

Next, the CPU 322 compares the signal A stored in the memory 310 with the threshold data (voltage value) stored in advance in the memory 324. Concretely, it is determined whether or not a voltage value of the store signal A is equal to or larger than a threshold value A (VthA) (Step S344). When, as a result of this determination, it is determined that the voltage value of the signal A is equal to or larger than the threshold value A (Step S344: Yes), this indicates that the autofluorescence 35e emitted from the viable particle 35 has entered the scattered light receiving device photo multiplier 90 and is detected. Then, a process of setting a detection flag indicating that the autofluorescence 35e is detected to ON is performed (Step S346). Incidentally, this detection flag (ON) is next transmitted as a flag signal to the analysis result outputting device 330.

On the other hand, when, as a result of these determinations, it is determined that the voltage value of the signal B is not equal to or larger than the threshold value B (Step S342: No), or it is determined that the voltage value of the signal A is not equal to or larger than the threshold value A (Step S344: No), a process of setting the detection flag to OFF (Step S348) is performed, which indicates that the autofluorescence 35e is not detected. Here, when the aforesaid scattered light detection flag is ON and the detection flag is OFF, a process of setting a non-viable detection flag indicating that the non-viable particle 37 which is not the viable particle 35 is detected to ON may be performed. Incidentally, this detection flag (OFF) is next transmitted as a flag signal to the analysis result outputting device 330. Further, the non-viable detection flag may be also transmitted.

The above-described analyzing process will be concretely described by using charts of the signal A and the signal B corresponding to the output signals output from the respective light receiving devices.

### [Examples of output signals from autofluorescence receiving device and scattered light receiving device]

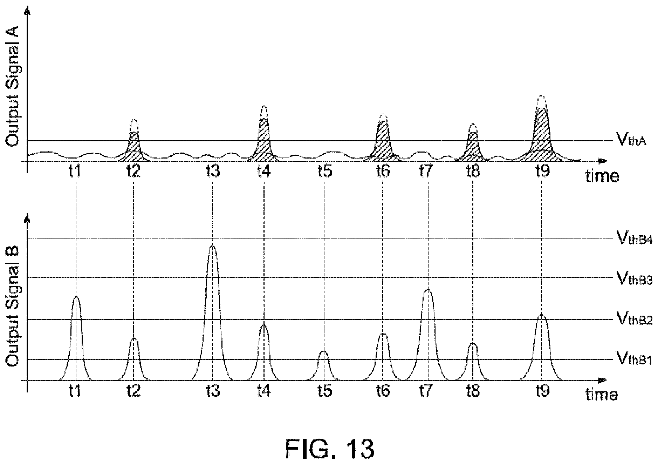

FIG. 13 is a chart illustrating examples of the output signals from the autofluorescence receiving device and the scattered light receiving device.

The signal on the upper side in FIG. 13 represents a time-variable distribution of the signal A corresponding to the detection signal output from the photo multiplier 90 being the autofluorescence receiving device, and the signal on the lower side in FIG. 13 represents a time-variable distribution of the signal B corresponding to the detection signal output from the photodiode 110 being the scattered light receiving device. Here, it is assumed that the distributions of the signal A and the signal B illustrated on the upper and lower sides are timing-adjusted distributions. Further, regarding the time on the horizontal axis, it is shown that the time passes in order of times t1, t2, t3, ...

For exmaple, if the voltage value of the signal B larger than the threshold value B (VthB (in the drawing, VthB1)) is input to the data processing part 300 at the time t1, the CPU 322 determines that the voltage value of the signal B is equal to or larger than the threshold value B (Step S342: Yes). That is, this indicates that the scattered light from the viable particle 35 or the non-viable particle 37 enters the scattered light receiving device photodiode 110 and is detected at t1.

Then, the CPU 322 compares the threshold value A (VthA) stored in advance in the memory 324 with the voltage value of the signal A (Step S344). Since the signal A at the time t1 is not a signal having a larger value than the threshold value A (Step S344: No), the signal B at the time t1 indicates the scattered light from the non-viable particle 37, and the detection flag is set to OFF (Step S348).

Next, at the time t2, the CPU 322 determines that the voltage value of the signal B is equal to or larger than the threshold value B (Step S342: Yes).

Then, the CPU 322 compares the threshold value A (VthA) with the voltage value of the signal A (Step S344), and as a result, the CPU 322 determines that the voltage value of the signal A is equal to or larger than the threshold value A (Step S344: Yes). Therefore, the signal A and the signal B at the time t2 indicate the auto fluorescence 35e and the scattered light from the viable particle 35, and the detection flag is set to ON (Step S346).

In the above-described manner, the result on whether or not the viable particle 35 exists is obtained in real time. Here, the intensities of the signal A and the signal B depend on amounts of the lights entering the autofluorescence receiving device photo multiplier 90 and the scattered light receiving device photodiode 110, and the intensity of the scattered light depends on the size of the viable particle 35 or the non-viable particle 37. Therefore, not only the presence/absence of the viable particle 35 is detected but also the size of the viable particle 35 or the non-viable particle 37 can be measured based on the intensities of the signal A and the signal B.

Here, it is assumed that a plurality of the threshold values B (VthB1, VthB2, VthB3, VthB4, ...) corresponding to the sizes (0.1 µm to 0.3 µm, 0.3 µm to 0.5 µm, 0.5 µm to 1.0 µm, ...) of the viable particles 35 are stored in the memory 324 in advance. For example, since the signal B at the time t2 has the intensity larger than VthB1 and smaller than VthB2, the size of the viable particle 35 is measured as 0.1 µm to 0.3 µm. Incidentally, the plural threshold values B corresponding to the sizes of the viable particles 35 (0.1 µm or larger, 0.2 µm or larger, ...) may be stored, and these threshold values B may be decided as desired.

In this example, the analyzing process S340 is performed for the data stored at the signal storage step S310, but instead of storing them, by comparing them with the threshold values B, A successively, the viable particle 35 or the non-viable particle 37 may be detected, and the peak of the signal B may be detected in real time, thereby finding particle size classification. Further, since the intensity of the signal A depending on the light amount of the autofluorescence 35e also corresponds to the kind and an activation state of the viable particle, these pieces of information may be also found by detecting the peak of the signal A.

In the above-described manner, based on the signal A and the signal B, it is possible to detect whether or not the viable particle 35 exists in real time and further to measure the size of the viable particle. When the detection flag is set to ON as a result of the detection on the presence/absence of the viable particle 35, the counting process of the viable particles 35 is next performed by the analysis result outputting process Step S400.

### [Analysis result outputting device (viable particle counting means)]

The analysis result outputting device 330 is a device which counts the number of the viable particles 35 derived from the analysis by the data analyzing device 320 and transmits the count value to the notifying part 400.

### [Analysis result outputting process]

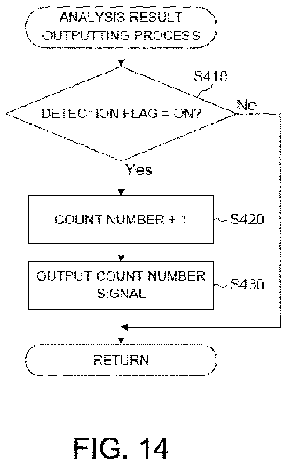

FIG. 14 is a flowchart illustrating a procedure example of the analysis result outputting process.

First, the analysis result outputting device 330 receives the flag signal (detection flag) from the data analyzing device 320. Then, it determines whether or not the detection flag is set to ON (Step S410). When as a result the detection flag is ON (Step S410: Yes), it determines that the viable particle 35 is detected, and increments the count number by 1 to calculate the count value (Step S420). Then, the count value (count number) is transmitted to the notifying part 400 (Step S430). Here, when a reset button (not illustrated) is pressed or when a start button (not illustrated) is pressed, a not-illustrated process of resetting the count number may be executed before Step S410. Further, based on the received flag signal (size flag), the number of the viable particles 35 may be counted for each size of the viable particles 35.

### [Notifying part]

The notifying part 400 is composed of, for exmaple, a display device 410 and a speaker 420.

### [Notifying process]

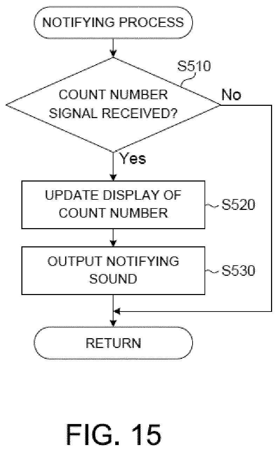

FIG. 15 is a flowchart illustrating a procedure example of the notifying process.

First, the notifying part 400 receives the count value transmitted by the analysis result outputting device 330 of the data processing part 300 (Step S510). Next, it displays, on the display device 410, the number of the detected viable particles 35 which is updated to the received count value (Step S520). Further, notifying sound is output from the speaker (Step S530). Here, the number of the detected viable particles 35 may be displayed on the display device 410 for each size of the viable particles 35. Another possible display form is to increment the count value by 1 in real time, or may be to display the updated count value a predetermined time later (for example, at five second interval).

An output manner of the notifying sound (the number of times the notifying sound is output, pitch of the notifying sound) may be changed according to a frequency with which the count value is incremented. Further, the output manner of the notifying sound may also be changed according to the size of the viable particle 35. For exmaple, the output manner may be such that, when the count value of the viable particles 35 with 0.5 µm to 1.0 µm in unit time is 1 to 9, simple-interval sound is output once such as "Pi!", when this count value is 10 to 99, simple-interval sound is output twice such as "Pi! Pi!", and when this count value is 100 or more, simple-interval sound is output three times such as "Pi! Pi! Pi!".

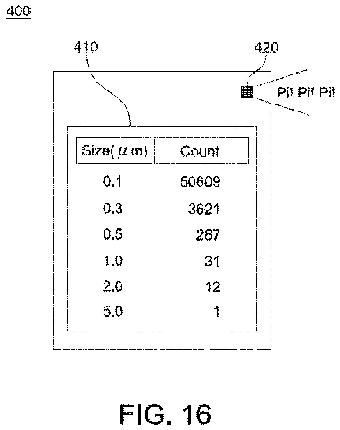

FIG. 16 is a view illustrating an exmaple of the display device and the speaker which notify the counting result of the viable particles 35. As the display device, there are provided a display panel 410 which notifies the counting result for each size of the viable particles 35 and a speaker 420 which notifies that the viable particle 35 is detected by means of sound. For example, the display panel 410 includes a display part for "Size (µm)" representing a reference of the size of the viable particles 35 and a display part for "Count" representing the number of the detected viable particles 35 corresponding to each size (count value). On the display part for "Size (µm)" representing the reference of the size of the viable particles 35, for example, six values "0.1", "0.3", "0.5", "1.0", "2.0", and "5.0" are displayed in advance. Regarding these values, "0.1" corresponds to a 0.1 µm to 0.3 µm size of the viable particles 35, "0.3" corresponds to a 0.3 µm to 0.5 µm size of the viable particles 35, "0.5" corresponds to a 0.5 µm to 1.0 µm size of the viable particles 35, "1.0" corresponds to a 1.0 µm to 2.0 µm size of the viable particles 35, "2.0" corresponds to a 2.0 µm to 5.0 µm size of the viable particles 35, and "5.0" corresponds to a 5.0 µm size of the viable particles 35 or more.

Therefore, it is shown that 50,605 pieces of the viable particles 35 whose size is 0.1 µm to 0.3 µm are counted, 3,621 pieces of the viable particles 35 whose size is 0.3 µm to 0.5 µm are counted, 287 pieces of the viable particles 35 whose size is 0.5 µm to 1.0 µm are counted, 31 pieces of the viable particles 35 whose size is 1.0 µm to 2.0 µm are counted, 12 pieces of the viable particles 35 whose size is 2.0 µm to 5.0 µm are counted, and 1 piece of the viable particle 35 whose size is 5.0 µm or larger is counted.

As described above, the notifying part 400 is capable of notifying the count value of the viable particles 35 in real time from the display device 410 and outputting the notifying sound from the speaker 420 when the viable particles 35 are detected. Incidentally, the notifying part 400 may be additionally provided with an external output terminal and may output the data to another device through the terminal.

Note that the autofluorescence selecting optical device of the light detecting system 1 is not limited to the long-pass filter 70 but may be formed by a dichroic mirror. For example, a dichroic mirror which transmits a light having a wavelength longer than the 490 nm cutoff wavelength and reflects a light with a wavelength shorter than the 490 nm cutoff wavelength may be provided. As a result, a light having a wavelength shorter than 490 nm (mainly the scattered light from the viable particle 35 or the non-viable particle 37) is received by the scattered light receiving device photodiode 110. On the other hand, a light having a wavelength longer than 490 nm (mainly the autofluorescence 35e from the viable particle 35e) is received by the autofluorescence receiving device photo multiplier tube 90.

Besides, regarding the scattered light selecting optical device and the autofluorescence selecting optical device of the light detecting system 1, instead of installing the autofluorescence selecting optical device on a subsequent stage of the scattered light selecting optical device, the scattered light selecting optical device and the autofluorescence selecting optical device may be installed in parallel so that optical paths of the scattered light and the autofluorescence become separate systems. In this case, a short-pass filter which transmits only a light whose wavelength is shorter than the 410 nm cutoff wavelength is used as the scattered light selecting optical device. Then, the condensing lens optical systems which gather the scattered light and the autofluorescence 35e from the flow cell, and the scattered light receiving device photodiode 110 and the autofluorescence receiving device 90 are installed in front of and at the back of the respective optical devices. Consequently, for example, owing to the scattered light selecting optical device (short-pass filter) installed at a 90 degree position (horizontal plane) from the optical axis of the laser light 31 and using 410 nm as a reference, the scattered light receiving device photodiode 110 can detect mainly the scattered light from the viable particle 35 or the non-viable particle 37, and owing to the autofluorescence selecting optical device (long-pass filter) installed at a 90 degree position (vertical plane) from the optical axis of the laser light 31 and using 490 nm as the cutoff wavelength, the autofluorescence receiving device photo multiplier 90 can detect mainly the autofluorescence 35e from the viable particle 35.

As described above, according to this embodiment, it is possible to improve counting accuracy of the viable particles 35. For this purpose, the detection of the autofluorescence 35e emitted from the viable particle 35 being a detection (measurement) target is used as an index. The emission of the autofluorescence 35e is caused by radiating the laser light 31 to a substance necessary for metabolism of life activity occurring in a living organism, such as riboflavin and NAD(P)H in the cell of the viable particle 35. Incidentally, the wavelength of the laser light 31 differs depending on the substance emitting the autofluorescence, and the optimum wavelength is used. Further, when the laser light 31 is radiated, the Raman-scattered light 33s from water and the scattered light from the viable particle 35 are emitted, besides the autofluorescence from the viable particle. Therefore, in this embodiment, the dichroic mirror and the long-pass filter are provided. Consequently, the scattered light from the viable particle 35 can be reflected by the dichroic mirror, and by the long-pass filter, the Raman-scattered light 33s by water can be reduced and the autofluorescence 35e from the viable particle 35 can be transmitted.

### [Case where dialysis fluid is detection target liquid]

Next, a case where a viable particle counter 77 handles a dialysis fluid as a liquid whose viable particles are detection targets will be concretely described. That is, a case where a dialysis fluid is used as a liquid made to flow in a target flow device 30 of the viable particle counter 77 will be concretely described.

Conventionally, in the manufacture of a dialysis fluid administered to a human body, the dialysis fluid is first treated to a state not causing a problem even when administered to a human body, by filtering out viable particles by a filter or the like. Then, regarding the manufactured dialysis fluid, it is inspected whether or not the viable particles exist in the dialysis fluid by an inspection such as a method of cultivation (official analytical method) or a fluorescent dye method. Further, in blood dialysis to a human body, a dialysis fluid is made to flow in a specific medical instrument and blood having undergone the blood dialysis there is administered to a human body. This flowing dialysis fluid also undergoes a filtration treatment to be manufactured and managed, and similarly undergoes a viable particle inspection.

In the inspection by the cultivation method, the viable particles in the dialysis fluid are cultivated on a bleeding ground under a condition of low temperature and a long period (several days to one week) and it is confirmed whether or not a colony is formed, whereby it is inspected whether or not the viable particles exist. Further, in the inspection by the florescent dye method, a specific dyeing reagent is dropped to the dialysis fluid, and after several-minute dyeing, light is radiated, followed by photographing by a CCD camera or the like, and the number of points emitting light is counted, whereby it is determined whether or not the viable particles exist and the number of the viable particles is counted (for example, Japanese Patent Application Laid-open No. 2007-300840: Patent Document 5).

Here, when the dialysis fluid inspection is performed by the fluorescent dye method in the prior art of Patent Document 5, every time the inspection is performed, it is necessary to move the dialysis fluid to a place different from a place where the blood dialysis is performed, perform the dyeing process by the specific dyeing reagent, and count the number of the viable particles after the photographing by the CCD camera. Further, it takes about twenty minutes to confirm the result of the inspection of the dialysis fluid. Therefore, it is not possible to perform the inspection in real time during the blood dialysis.

Therefore, a system regarding dialysis fluid inspection using the aforesaid viable particle counter 77 will be described. Concretely, a system which is capable of inspecting a dialysis fluid administered to a human body in real time, detecting the viable particles in the dialysis fluid to count the number thereof, and notifying the result at the site based on a desired condition will be described.

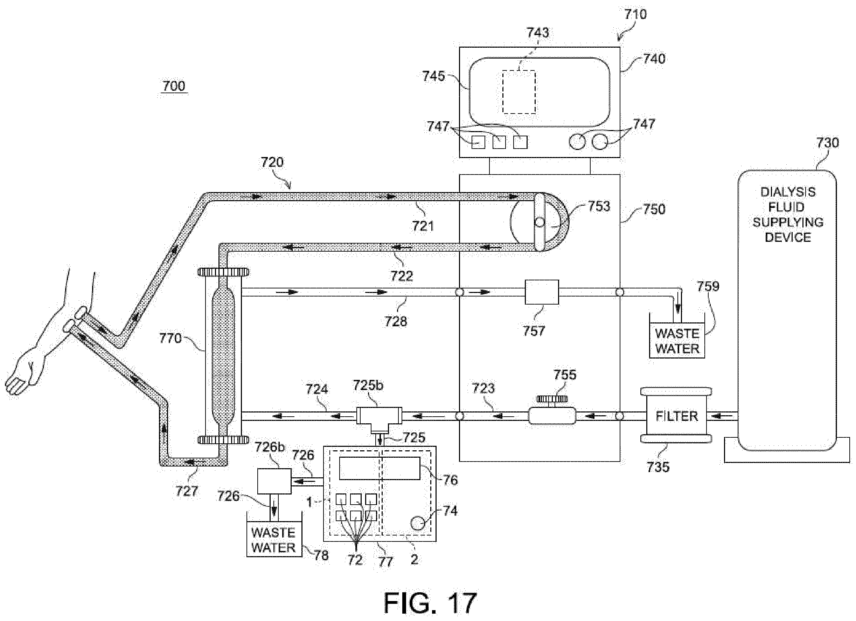

FIG. 17 is an explanatory diagram of the system relating to the dialysis fluid inspection in the blood dialysis.

As illustrated in FIG. 17, the blood dialysis of a patient is executed by a blood dialyzing device 700, and the aforesaid viable particle counter 77 executes the determination on whether or not the viable particles exist in the dialysis fluid used in the blood dialysis and the counting of the number of the viable particles. The blood dialyzing device 700 will be concretely described. Note that the application of the present invention is not limited to the dialysis fluid in the blood dialyzing device but may be a dialysis fluid in a blood filtering device.

### [Blood dialyzing device]

The blood dialyzing device 700 includes: a dialysis monitoring device 710 which monitors and adjusts patient information, the flow of the blood and the dialysis fluid, and so on; a distributing circuit 720 in which the blood and the dialysis fluid flow; a blood purifier 770 which diffuses deficient matter from the dialysis fluid to the blood while expelling waste products in the blood; and a dialysis fluid supplying device 730 which supplies the dialysis fluid.

### [Dialysis monitoring device]

The dialysis monitoring device 710 includes a controlling and monitoring part 740 and a distribution adjusting part 750. The controlling and monitoring part 740 is a device which controls and monitors the blood dialysis in general, and includes a controlling and monitoring device 743, a display part 745 (notifying means), and an operation part 747. The controlling and monitoring device 743 performs the distribution adjustment of the blood collected from the patient and the blood administered after the blood dialysis, the adjustment of temperature and distribution of the dialysis fluid sent out from the dialysis fluid supplying device 730 to the blood purifier 770, the monitoring of a body condition (for example, blood pressure, electrocardiogram information, and so on) of the patient, the distribution and monitoring of the dialysis fluid before and after the blood dialysis, and so on. The display part 745 (notifying means) displays various kinds of information, a progress status, input data, and so on. The operation part 747 accepts an operation from the outside (for example, a medical worker).

Concretely, the controlling and monitoring device 743 is a device composed of a CPU which executes various kinds of processing, a ROM storing the processing contents (program and data), a RAM storing various kinds of information, and so on. Further, the display part 745 is a display and displays information output from the controlling and monitoring device 743. Further, the operation part 747 is composed of, for example, a plurality of input buttons, and is capable of accepting various kinds of processing and accepting input and output of various kinds of information when a predetermined operation button is pressed by a medical worker. Further, an external connection terminal, though not illustrated, which accepts a control signal from the outside (central controlling device) and outputs various kinds of information may be provided.

### [Distribution adjusting part]

The distribution adjusting part 750 includes a blood pump 753, a dialysis fluid regulating valve 755, and a discharging device 757. The blood pump 753 adjusts a flow rate of the blood collected from the patient and circulates the blood. The dialysis fluid regulating valve 755 adjusts a flow rate of the dialysis fluid supplied from the dialysis fluid supplying device 730. The discharging device 757 discharges the dialysis fluid discharged from the blood purifier 770. These blood pump 753, dialysis fluid regulating valve 755, and discharging device 757 are controlled by the aforesaid controlling and monitoring device 743. Incidentally, the dialysis fluid discharged to the outside of the dialysis monitoring device 710 from the discharging device 757 is made to flow to a waste water disposal part 759 and thereafter undergoes waste water disposal.

### [Distributing circuit]

The distributing circuit 720 includes blood circuits 721, 722, 727 and dialysis fluid circuits 723, 724, 725, 727, 728.

### [Blood circuits]

The blood circuit 721 connects an artery of the patient and a blood inlet port of the blood pump 753 and makes the blood collected from the patient flow to the blood pump 753. The blood circuit 722 connects a blood output port and a blood inlet port of the blood purifier 770 and makes the blood sent out from the blood pump 753 flow to the blood purifier 770. The blood circuit 727 connects a blood outlet port of the blood purifier 770 and a vein of the patient and administers the blood having undergone the blood dialysis by the blood purifier 770 to the vein of the patient. A flow rate of the blood flowing in the blood circuits 721, 722, 727 is adjusted by the blood pump 753, and for example, the blood at a flow rate of about 200 ml/min circulates.

### [Dialysis fluid circuits (liquid distributing means, liquid distributing step)]

The dialysis fluid circuit 723 has the dialysis fluid supplied from the dialysis fluid supplying device 730 flow therein. Incidentally, in the middle of the dialysis fluid circuit 723, the valve 755 for dialysis which adjusts a flow rate of the dialysis fluid is provided. Before being made to flow into a dialysis fluid inlet port of the blood purifier 770, the dialysis fluid flowing in the dialysis fluid circuit 723 is made to branch off by a dialysis fluid divider 725b (liquid dividing means) in order for the viable particles in the dialysis fluid to be counted. Concretely, after branching off, part of the dialysis fluid flowing in the dialysis fluid circuit 723 is made to flow to the dialysis fluid circuit 725 to be thereafter sent to the viable particle counter 77. On the other hand, most of the dialysis fluid flowing in the dialysis fluid circuit 723 is made to flow to the dialysis fluid circuit 724 to be made to flow into the dialysis fluid inlet port of the blood purifier 770. The dialysis fluid circuit 726 makes the dialysis fluid having undergone the detecting and counting processes of the viable particles in the viable particle counter 77 flow to a waste water disposal device 78, and the dialysis fluid undergoes a waste water disposal process in the waste water disposal device 78. The dialysis fluid circuit 728 makes the dialysis fluid sent out from the dialysis fluid port of the blood purifier 770 flow therein. Incidentally, in the middle of the dialysis fluid circuit 728, the discharging device 757 which discharges the dialysis fluid to the waste water disposal device 759 is provided. After the flow rate of the dialysis fluid made to flow in these dialysis fluid circuits 723, 724, 725, 726, 728 is adjusted by the dialysis fluid regulating valve 755, the dialysis fluid is made to flow into the blood purifier 770. For example, the dialysis fluid at a flow rate of about 500 ml/min is made to flow to the blood purifier 770 and the viable particle counter 77, and the dialysis fluid at a flow rate of about 10 ml/min is made to flow into the viable particle counter 77.

### [Dialysis fluid supplying device]

The dialysis fluid supplying device 730 is a device which supplies the dialysis fluid, and concretely is a storage tank. For example, the dialysis fluid is stored in the tank, and the dialysis fluid can be supplied to the blood purifier 770 and the viable particle counter 77 by the dialysis fluid regulating valve 755 in the dialysis monitoring device 710 being operated. Further, a filter 735 is installed between the dialysis fluid supplying device 730 and the blood purifier 770, and there, the final purification of the dialysis fluid before it flows into the blood purifier 770 is performed.

### [Blood purifier]

The blood purifier 770 is, for example, a device which purifies the blood collected from the patient and concretely is a blood dialyzer. Incidentally, when it is a blood filtering device, a blood filter (hemofilter) is used. In this embodiment, a description will be given assuming that the blood dialyzer 770 is used as the blood purifier.

The dialyzer 770 includes the blood inlet port, the blood outlet port, the dialysis fluid inlet port, and the dialysis fluid outlet port. Connection destinations of the inlet ports and the outlet ports of the dialyzer 770 are as follows. The blood inlet port is connected to the blood circuit 722, the blood outlet port is connected to the blood circuit 727, the dialysis fluid inlet port is connected to the dialysis fluid circuit 724, and the dialysis fluid outlet port is connected to the dialysis fluid circuit 728. Further, the dialyzer 770 includes a plurality of (for example, several thousand) hollow fiber membranes allowing the blood to flow therein. Therefore, the blood flowing into the dialyzer 770 from the blood inlet port connected to the blood circuit 722 branches off into the several thousand hollow fiber membranes. Thereafter, the blood having undergone the blood dialysis via the hollow fiber membranes is made to flow out to the blood circuit 727 from the blood outlet port. On the other hand, the dialysis fluid flowing into the dialyzer 770 from the dialysis fluid inlet port connected to the dialysis fluid circuit 724 is made to flow in a direction opposite that of the blood to peripheries of the plural hollow fiber membranes. Thereafter, the dialysis fluid having undergone the blood dialysis via the hollow fiber membranes is made to flow out to the dialysis fluid circuit 728 from the dialysis fluid outlet port. Inside the dialyzer 770, the waste products in the blood move into the dialysis fluid flowing outside the hollow fiber membranes and the deficient components in the blood move into the blood through the hollow fiber membranes from the dialysis fluid.

### [Viable particle counter for dialysis]

The viable particle counter 77 supplied with the dialysis fluid from the dialysis fluid supplying device 730 may further include operation parts 72, 74 and a notification display 76 (notifying means) in addition to a light detecting system 1 and an autofluorescence counting system 2 as described above. The light detecting system 1 is an apparatus which radiates a light to a target (dialysis fluid) to detect a scattered light and an autofluorescence from the target. The autofluorescence counting system 2 is an apparatus which counts the number of the autofluorescences based on signals output from the light detecting system 1. The operation parts 72, 74 are composed of, for example, a plurality of kinds of buttons 72 and a controller 74 respectively, and are capable of accepting an operation of the viable particle counter 77 by a medical worker. Further, the notification display 76 is capable of displaying, for example, input information, operation information, and so on besides the aforesaid counting result notified by the notifying part 400.

Concretely, in the viable particle counter 77, the dialysis fluid supplied from the dialysis fluid supplying device 730 first flows in the target flow device 30, and a laser light having a 375 nm to 420 nm wavelength easily exciting riboflavin is radiated to the flowing dialysis fluid from a light emitting device 10. Thereafter, as described above (refer to FIG. 1), due to an interaction of the radiated laser light with water (water molecules) of the dialysis fluid flowing in the target flow device 30 and with the target (a viable particle 35 or a non-viable particle 37), a scattered light, a Raman-scattered light, and the autofluorescence are emitted to the surroundings. Then, these lights pass through a plurality of condensing lens systems 40, 80, 100 and wavelength selecting optical devices (scattered light selecting optical device 60, autofluorescence selecting optical device 70) to be detected by light receiving devices (autofluorescence receiving device 90, scattered light receiving device 110). Further, as described above (refer to FIG. 8 and FIG. 9), as for the Raman-scattered light emitted from the water of the dialysis fluid and the autofluorescence, most of the Raman-scattered light by the water is cut off by the long-pass filter 70 using a cutoff wavelength (for exmaple, 490 nm) as the reference, while the autofluorescence is transmitted, for instance. Thereafter, detection signals of the scattered light selecting optical device 60 and the autofluorescence selecting optical device 70 are transmitted to the autofluorescence counting system 2, and based on the transmitted data, the presence/absence of the viable particle 35 is confirmed in real time, and the viable particles 35 are counted for each particle size. Then, finally, the counting result is displayed on the notification display 6 and output to the outside.

Incidentally, it is preferable to provide a suction pump or a flow rate regulating valve 726b inside the viable particle counter 77 or the dialysis fluid circuit 726. For example, when the adjustment of a supply amount of the dialysis fluid by the dialysis fluid supplying device 730 is not sufficient, providing the suction pump or the flow rate regulating valve 726b makes it possible to facilitate the adjustment.

As described above, according to this embodiment, by using the viable particle counter 77, it is possible to determine in real time whether or not the viable particle 35 exists regarding the dialysis fluid. Note that in the determination on the presence/absence of the viable particles regarding the dialysis fluid as well, the detection of the autofluorescence 35e is used as an index. The autofluorescence 35e is emitted from an autofluorescent substance required for metabolism of life activity occurring in a living organism, such as riboflavin and NAD(P)H in a cell of the viable particle 35 being the detection (measurement) target. When as a result of the counting by the viable particle counter 77, it is confirmed that a prescribed number of the viable particles 35 or more exist in the dialysis fluid for which the counting is performed, the result is notified to the outside from a notifying means provided in the viable particle counter 77. For example, it is possible to output notifying sound from a speaker and perform the display on the display 745 of the dialysis monitoring device 710 or a central monitoring and control device 640. This makes it possible to take a quick measure to the dialysis fluid before the contamination by the viable particles progresses and to reduce an influence on the patient to the minimum.

Next, a dialysis fluid monitoring system including this viable particle counter 77 will be described.

### [Dialysis fluid monitoring system including viable particle counter]

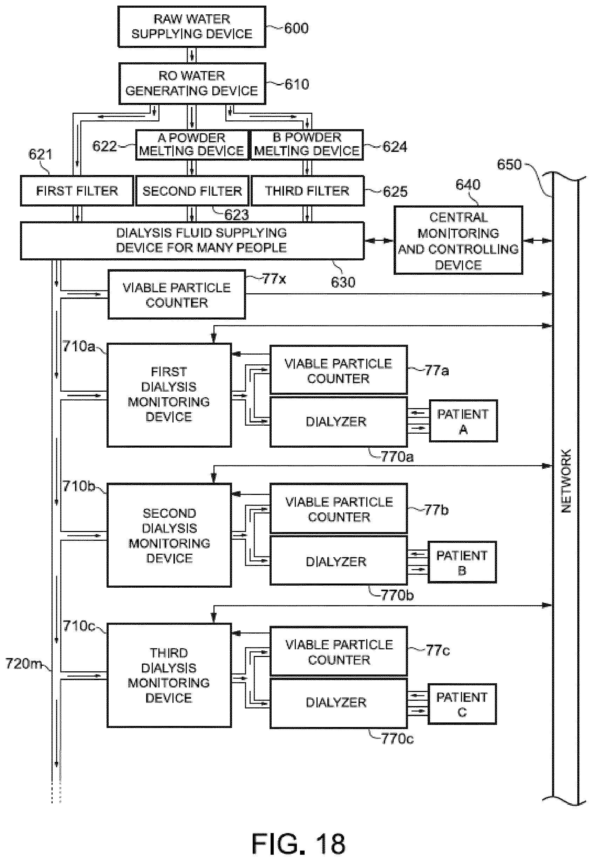

FIG. 18 is an explanatory diagram of an artificial dialysis device system for many people.

As illustrated in FIG. 18, the artificial dialysis device system for many people includes a system which performs blood dialysis and a dialysis fluid monitoring system. The system which performs blood dialysis performs blood dialyses of a plurality of patients at the same time by using one dialysis fluid supplying device 630 for many people. The dialysis fluid monitoring system determines whether or not viable particles exist in the dialysis fluid by viable particle counters 77 installed at various places.

The dialysis fluid used by the artificial dialysis device system for many people is manufactured through a plurality of steps. First, a RO water generating device 610 removes ions and other organic matter from water of a raw water supplying device 600 (for example, a tap water), so that purified RO water (water purified by a reverse osmosis membrane (RO membrane), what is called pure water) is manufactured. Then, by using the RO water, an A diluting solution for dialysis is manufactured by an A powder melting device 622 and a B diluting solution for dialysis is manufactured by a B powder melting device 624. Finally, the RO water purified by a first filter 621, the A diluting solution for dialysis purified by a second filter 623, and the B diluting solution for dialysis purified by a third filter 625 are mixed, whereby the dialysis fluid is manufactured. This manufactured dialysis fluid is stored in the dialysis fluid supplying device 630 for many people (dialysis fluid supplying means for many people) (dialysis fluid supplying step for many people).

The dialysis fluid stored in the dialysis fluid supplying device 630 for many people, for example, flows in a pipe 720m for dialysis (liquid distributing means for many people) to branch off to a first dialysis monitoring device 710a, a second dialysis monitoring device 710b, a third dialysis monitoring device 710c, ... (liquid distributing step for many people). Then, the dialysis fluid flows into dialyzers 770a, 770b, 770c connected to the respective dialysis monitoring devices 710a, 710b, 710c. Further, bloods collected from patients A, B, C undergo blood dialysis in the dialyzers 770a, 770b, 770c, and thereafter the purified bloods are administered to the patients.

Incidentally, it is assumed here that the blood dialyses for the plural patients are monitored by the central monitoring and controlling device 640 (viable particle determination result collecting means for many people). Concretely, the blood dialyses are monitored by transmitting, in real time, pieces of information regarding the blood dialyses performed in the respective dialysis monitoring devices 710a, 710b, 710c to the central monitoring and controlling device 640 via a network 650 (viable particle determination result collecting step for many people).

Further, in this example, the viable particle counters 77 installed at various places determine whether or not the viable particles exist in the dialysis fluid. For example, it is determined whether or not the viable particles exist in the dialysis fluid before the bloods having undergone the blood dialysis using the dialysis fluid are administered to the patients A, B, C, that is, before the dialysis fluid is made to flow into the dialyzers 770a, 770b, 770c. This determination is made possible by providing viable particle counters 77a, 77b, 77c between the dialysis monitoring devices 710a, 710b, 710c and the dialyzers 770a, 770b, 770c. Further, the counting results of the viable particle counters 77a, 77b, 77c are transmitted to the respective dialysis monitoring devices 710a, 710b, 710c and then transmitted from the dialysis monitoring devices 710a, 710b, 710c to the central monitoring and controlling device 640 via the network 650. Therefore, when, as a result of the counting by any of the viable particle counters 77, it is confirmed that a prescribed number of the viable particles or more exist in the dialysis fluid for which the counting is performed, a notifying means provided in the viable particle counter 77 notifies the confirmation result to the outside. For example, notifying sound is output from a speaker, or the display indicating a state of the dialysis fluid is made on the display 745 (notifying means) of the dialysis monitoring device 710 or the central monitoring and controlling device 640. Consequently, it is possible to confirm that the viable particles exist in the dialysis fluid in real time at a site where the blood dialysis is executed. As a result, it is possible to take a quick measure to the dialysis fluid before the contamination by the viable particles progresses, which can reduce an influence on the patient to the minimum.

Besides, the viable particle counter 77 may be installed in the dialysis fluid supplying device 630 for many people and the water distribution pipe 720m for dialysis (dialysis fluid distributing means for many people). Consequently, it is possible to directly inspect the dialysis fluid distributed from the dialysis fluid supplying device 630 for many people. Further, the viable particle counters 77, not illustrated, may be provided in front of and at the back of the RO water generating device 610, the A powder melting device 622, the B powder melting device 624, the first filter 621, the second filter 623, and the third filter 625. Consequently, it is possible to confirm which of the devices or which of the pipes between the device and the device is contaminated by the viable particles.

### [Case where purified water is detection target liquid]

Next, a case, not part of the present invention, where water having undergone purification treatment is handled as a liquid in which viable particles are to be detected by a viable particle counter 77 will be concretely described. That is, water having undergone purification treatment is made to flow in a target flow device 30. Besides, by additionally providing a viable particle counter 77, water under the purification treatment may be made to flow in its target flow device 30.

Conventionally, water sent from a purified water basin provided in a water purification plant, an aquarium, a lake or a marsh, a garden, or the like has been purified by filtration, disinfection, or the like in order to eliminate and kill viable particles contained in the water for purification. Further, regarding this purified water, it is inspected by a turbidimeter, a fine particle counter, or the like whether or not sterilizing purification of the viable particles has been performed efficiently.

For example, in the inspection by the turbidimeter, turbidity is calculated, and from this turbidity, a probability that viable particles in water are eliminated by sterilizing purification is calculated. A concrete inspection method is performed by radiating a light to the water containing particles (viable particles or non-viable particles), and comparing the light radiated from a light source and a scattered light received by a light receiving device, or detecting an interference light due to the particles. Further, by using the fine particle counter, it is confirmed whether or not a specific pathogenic worm (for example, cryptosporidium) in water is eliminated by the sterilizing purification (for example, refer to Japanese Patent Application Laid-open No. 2009-673: Patent Document 6). In a concrete confirmation method, a fluorescent labeled antibody easily bonding with the pathogenic worm being the detection target is mixed with the purified water, and it is confirmed whether or not the water is purified by sterilization based on whether or not a fluorescence light emitted from the detection target is detected.

Here, according to the prior art of Patent Document 6, the real-time inspection of the water having undergone the purification treatment is not possible. This is because, as the detection method, an antigen-antibody reaction is utilized by using chemicals such as the fluorescent labeled antibody. For example, it takes twenty minutes to thirty minutes at 37°C or one hour or more at room temperature for the antigen-antibody reaction between cryptosporidium and the fluorescent labeled antibody to end. Further, since an amount of the detection target is only a little due to the purification treatment, a large amount of the fluorescent labeled antibody is required in order to easily cause the antigen-antibody reaction. In addition, preliminary arrangements for the inspection such as filtering the water having undergone the purification treatment by using a filter with a 3 µ pore size, capturing and condensing large fine particles containing the cryptosporidium on a surface of the filter, and thereafter using the fluorescent labeled antibody are required in advance. Here, when the inspection (detection) of viable particles other than cryptosporidium is performed, it is necessary to use another fluorescent labeled antibody easily undergoing an antigen-antibody reaction with the viable particles. Therefore, the inspection method utilizing the antigen-antibody reaction with the viable particles has problems that it requires a predetermined time to confirm the inspection result, a plurality of kinds of the fluorescent labeled antibodies have to be prepared, and the purified water has to be condensed or a large amount of the fluorescent labeled antibody has to be prepared.

Therefore, in this embodiment, a viable particle inspection method for purified water which uses, instead of the inspection method utilizing the antigen-antibody reaction with the viable particles, the aforesaid viable particle counter 77 is used. The viable particle inspection of the purified water using the viable particle counter 77 is capable of the real-time inspection, detecting the viable particles in the purified water to count the number thereof, and notifying an abnormal state, if any. Next, a system relating to the viable particle inspection of the purified water using the viable particle counter will be concretely described.

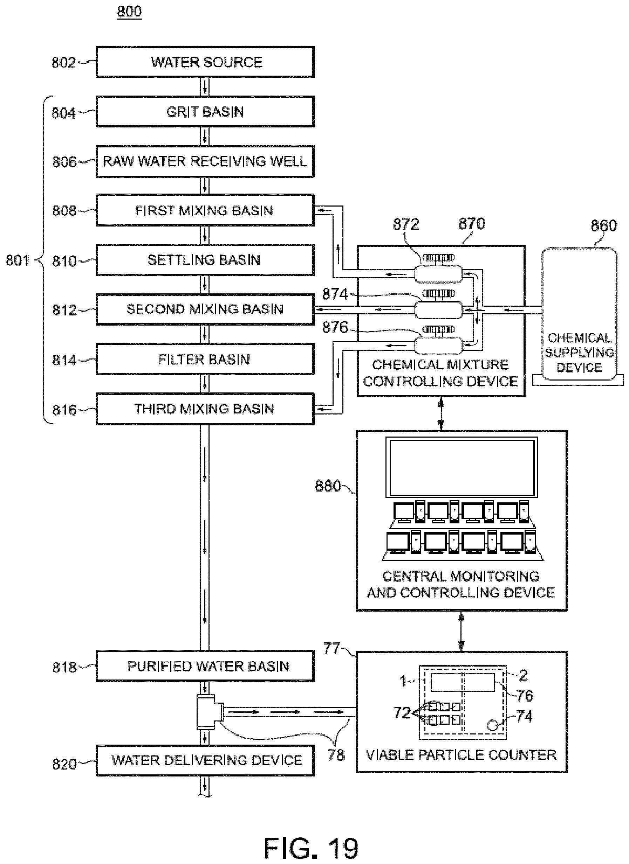

FIG. 19 is an explanatory diagram of a system relating to the inspection of viable particles in purified water in a water purification plant.

As illustrated in FIG. 19, regarding the purified water having undergone the purification treatment by a water purifying system 800 in the water purification plant, a viable particle counter 77 inspects (detects) whether or not the viable particles exist. Further, the number of the viable particles is counted. The water purifying system 800 will be concretely described.

### [Water purifying system]

The water purifying system 800 is composed of a plurality of purifying basins 801 (purifying means) where the purification treatment is performed, a purified water basin 818 storing the manufactured purified water in the water purification plant, a water delivering device 820 which delivers the manufactured purified water, a chemical supplying device 860, a chemical mixture controlling device 870, and a central monitoring and controlling device 880. Further, the plural purifying basins 801 (purifying means) include, for example, a grit basin 804, a raw water receiving well 806, a first mixing basin 808, a settling basin 810, a second mixing basin 812, a filter basin 814, a third mixing basin 816, and so on. The water purifying system purifies raw water taken from a water source 802 by these constituent elements to manufacture the purified water. Hereinafter, the constituent elements will be concretely described.

### [Central monitoring and controlling device (central monitoring and controlling means)]

The central monitoring and controlling device 880 is composed of a plurality of computers and is a device which monitors and controls the whole purification treatment of the water purifying system 800. Concretely, the central monitoring and controlling device 880 performs the control for purifying the raw water to manufacture clean purified water, such as controlling the purification treatment, the adjustment of a water amount, the monitoring of water quality, water delivery, and so on. Here, the purification treatment means to purify the water in the purifying basins 804 to 816 and so on. Further, the adjustment of a water amount and the monitoring of water quality mean the adjustment of a water amount and the monitoring of a purification state and so on at the time of the water purification performed in the purifying basins 804 to 816 and so on. Further, the water delivery means to deliver the water having undergone the purification treatment to the next purifying basin. Incidentally, the manufactured purified water is once stored in the purified water basin 818 and is delivered to of respective areas by the water delivering device 820. Further, the central monitoring and controlling device 880 also controls the chemical mixture controlling device 870. The chemical mixture controlling device 870 adjusts an injection amount of chemicals used when the water is disinfected in the first mixing basin 808, the second mixing basin 812, and the third mixing basin 816. In these first mixing basin 808, second mixing basin 812, and third mixing basin 816, the raw water taken from the water source 802 is purified step by step, and through these mixing basins, the purified water is finally manufactured. Hereinafter, concrete purification treatments in the constituent elements will be described.

### [Grit Basin]

In the grit basin 804, the raw water taken from the water source 802 is pooled. For example, when the water source 802 is a river, the taken raw water contains large dusts, sands, soil, and so on. Therefore, while the raw water is pooled in the grit basin 804, the large dusts are removed, and the sands and soil are settled in a bottom of the grit basin 804, resulting in a certain degree of purification. The water from which the large dusts, sands, and soil are eliminated is next sent to the raw water receiving well 806 by an intake pump or the like.

### [Raw water receiving well]

In the raw water receiving well 806, the water sent from the grit basin 804 is pooled. Here, when raw waters are taken from a plurality of water sources 802, the waters pooled in a plurality of grit basins 804 corresponding to the respective water sources are combined by this raw water receiving well 806. Then, this raw water receiving well 806 adjusts a flow rate of the water that is to be sent to the first mixing basin 808 based on a level and an amount of the pumped-up water.

### [First mixing basin]

In the first mixing basin 808, the water sent mainly from the raw water receiving well 806 and a flocculant are mixed. Incidentally, sodium hypochlorite (hereinafter referred to as chlorine) used for sterilization or the like is sometimes injected. The flocculant is, for example, poly aluminum chloride or the like. Using a stirring device for the mixture with the flocculant facilitates mixing fine sands, soil, and so on with the flocculant. Further, by adjusting a stirring speed, they are flocculated (turned into a lump) separately for each kind such as small contaminants, turbidity components, and so on. The water mixed with the flocculant is next sent to the settling basin 810.

### [Settling basin]

In the settling basin 810, the small contaminants and the turbidity components contained in the water are flocculated (turned into a lump) by the flocculant mixed in the first mixing basin 808, to settle. The small contaminants include dusts, mud, organic matter, plankton, and so on. The water from which the lumped deposits are removed or a supernatant of the water pooled in the settling basin 810 is next sent to the second mixing basin 812.

### [Second mixing basin]

In the second mixing basin 812, the water sent from the settling basin 810 is supplied with, for example, chlorine. The chlorine is supplied from the chemical supplying device 860 via the chemical mixture controlling device 870. Consequently, the viable particles contained in the water pooled in the second mixing basin 812 reduce. The chlorinated water is next sent to the filter basin 814.

### [Filter basin]

In the filter basin 814, the water sent from the second mixing basin 812 passes through sands and gravels, and further fine dusts, turbidity components, and so on are filtered out. The filtered water is next sent to the third mixing basin 816.

### [Third mixing basin]

In the third mixing basin 816, the water sent from the filter basin 814 is chlorinated by the chlorine being injected again to this water. This chlorine is also supplied from the chemical supplying device 860 via the chemical mixture controlling device 870. Therefore, the viable particles contained in the water pooled in the third mixing basin 816 further reduce. Incidentally, the chlorination in the third mixing basin 816 is regarded as an important purification treatment. This is done in order to keep safety of the purified water until it is sent to an end (for example, a water tap of a house). Therefore, the chlorine with an amount large enough to eliminate the viable particles (for example, cryptosporidium or the like) liable to affect a human body is injected. Finally, the chlorinated purified water is next sent to the purified water basin 818.

### [Purified water basin]

In the purified water basin 818, the purified water sent from the third mixing basin 816 is pooled. Here, the quality of the purified water manufactured by the purification treatments so far is examined. In this embodiment, regarding the purified water pooled in the purified water basin 818, the viable particle counter 77 determines whether or not the viable particles exist in the purified water, and at the same time counts the number of the viable particles.

### [Water delivering device (water delivering means)]

The water delivering device 820 is, for example, a pressurizing-type water pump 820 and is a device which delivers the purified water pooled in the purified water basin to distribution basins in various areas through water pipes.

As described above, the water purifying system 800 purifies the raw water taken from the water source 802 by the plural purification treatments in the plural purifying basins 804 to 816. Then, the purified water manufactured through the purification is delivered from the purified water basin 818 by the water delivering device 820. Here, the chlorination in the purification by the water purifying system 800 will be concretely described.

### [Chemical supplying device (chemical supplying means)]

The chemical supplying device 860 is, for example, a storage tank and stores the sodium hypochlorite (chlorine) used at the time of the chlorination. In the illustrated example, it branches off from the single storage tank 860 into the first mixing basin 808, the second mixing basin 812, and the third mixing basin 816, where the sterilization and so on are performed, but the chemical supplying device 860 may be a device capable of further supplying the flocculant or the like, or the storage tank 860 may be provided per each of the purifying basins 808, 812, 816. The chlorine or the like supplied from the storage tank 860 is supplied to the purifying basins 808, 812, 816 via water pipes, and the chemical mixture controlling device 870 provided in the middle of the water pipes adjusts a flow rate of the injected chlorine.

### [Chemical mixture controlling device (chemical injection amount controlling means, chemical injecting means)]

The chemical mixture controlling device 870 is provided with regulating valves which adjust flow rates of the chlorine or the like supplied from the storage tank 860 via the pipes.

For example, a regulating valve 872 is connected to the water pipe between the storage tank 860 and the first mixing basin 808, a regulating valve 874 is connected to the water pipe between the storage tank 860 and the second mixing basin 812, and a regulating valve 876 is connected to the water pipe between the storage tank 860 and the third mixing basin. The regulating valves 872, 874, 876 adjust the flow rates of the chlorine or the like flowing in the water pipes. Incidentally, the adjustment by each of the regulating valves 872, 874, 876 is performed based on an instruction from the central monitoring and controlling device 880.

In the above-described manner, the chlorine is injected to the purifying basins 808, 812, 816, so that the chlorination is performed. Further, the chlorinated water is sent to the purified water basin 818. The purified water sent to the purified water basin 818 is sent by a dividing device 78 to the viable particle counter 77, where it is determined whether or not the viable particles exist in the purified water.

### [Dividing device (dividing means)]

The dividing device 78 is composed of, for example, a water pipe which makes the purified water from the purified water basin 818 branch off to lead the branching purified water to the viable particle counter 77, and when necessary, a suction pipe (not illustrated), a flow rate regulating valve (not illustrated) adjusting a flow rate in the water pipe, and so on. Then, the purified water inspected by the viable particle counter 77 is discharged from a water pipe for discharge. Incidentally, when the suction pump and the flow rate regulating valve are necessary, they are preferably provided in the discharge-side water pipe of the viable particle counter 77. For example, when the purified water cannot be stably led to the viable particle counter 77, this can be easily adjusted.

### [Viable particle counter for purified water]

The viable particle counter 77 supplied with the purified water from the dividing device 78 may further include operation parts 72, 74 and a notification display 76 (notifying means) besides a light detecting system 1 and an autofluorescence counting system 2 as described above. As described above, the light detecting system 1 is an apparatus which radiates a light to the target (purified water) to detect a scattered light and an autofluorescence from the target. The autofluorescence counting system 2 is an apparatus which counts the number of the autofluorescences based on signals output from the light detecting system 1. The operation parts 72, 74 are composed of, for example, a plurality of kinds of buttons 72 and a controller 74 and are capable of accepting an operation of the viable particle counter 77 by a purified water manager or the like. Further, the notification display 76 is capable of displaying, for example, input information, operation information, and so on in addition to the counting result notified by the aforesaid notifying part 400.

Concretely, in the viable particle counter 77, the purified water supplied from the dividing device 78 first flows in the target flow device 30. Then, a laser light having a 375 nm to 420 nm wavelength easily exciting riboflavin is radiated to the flowing purified water from a light emitting device 10 of the viable particle counter 77. Here, possible viable particles contained in the purified water are phytoplanktons besides bacteria, yeast, and mold described above. In order to detect whether or not the phytoplanktons exist in the purified water, the detection of an autofluorescence 35e of chlorophyll or the like of a substance existing in their cells serves as an index. An excitation wavelength spectrum of the chlorophyll presents a distribution having a peak at an about 430 nm wavelength, and therefore, the wavelength of the laser light from the light emitting device 10 is suitably 400 nm to 450 nm.

Thereafter, in the viable particle counter 77, a scattered light, a Raman-scattered light, and the autofluorescence are emitted to the surroundings due to an interaction of the radiated laser light with the purified water (water molecules) flowing in the target flow device 30 and with the target (the viable particle 35 or the non-viable particle 37), as described above (refer to FIG. 1). Then, these lights pass through a plurality of condensing lens systems 40, 80, 100 and wavelength selecting optical devices (scattered light selecting optical device 60, autofluorescence selecting optical device 70) to be detected by light receiving devices (autofluorescence receiving device 90, scattered light receiving device 110). Further, as described above (refer to FIG. 8 and FIG. 9), regarding the Raman-scattered light and the autofluorescence emitted from the purified water, most of the Raman-scattered light by the water is cut off by the long-pass filter 70 using a cutoff wavelength (for example, 490 nm) as its reference, for exmaple, while the autofluorescence is transmitted. Incidentally, when chlorophyll is used as an index, the cutoff wavelength of the long-pass filter or the like is suitably about 600 nm. This is because an autofluorescence spectrum of the chlorophyll presents a distribution having a peak at about 650 nm, and it is necessary to transmit lights having these wavelengths. Thereafter, detection signals of the scattered light selecting optical device 60 and the autofluorescence selecting optical device 70 are transmitted to the autofluorescence counting system 2. In the autofluorescence counting system 2, it is confirmed in real time whether or not the viable particles 35 exist based on the transmitted data, and the viable particles 35 are counted for each particle size. Then, finally, the counting result is displayed by the notification display 76 or output to the outside.

Note that the autofluorescence selecting optical device of the light detecting system 1 is not limited to the long-pass filter 70 and may be formed by a dichroic mirror. For example, a dichroic mirror that transmits a light having a wavelength longer than the 490 nm (600 nm) cutoff wavelength and reflects a light having a wavelength shorter than the 490 nm (600 nm) cutoff wavelength may be provided. By providing such a dichroic mirror, a light having a wavelength shorter than 490 nm (mainly the scattered light from the viable particle 35 or the non-viable particle 37) is received by the scattered light receiving device photodiode 110, and a light having a wavelength longer than 490 nm (600 nm) (mainly the autofluorescence 35e from the viable particle 35) is received by the autofluorescence receiving device photo multiplier tube 90.

Besides, regarding the scattered light selecting optical device and the autofluorescence selecting optical device of the light detecting system 1, the autofluorescence selecting optical device need not be installed on a subsequent stage of the scattered light selecting optical device. Concretely, the scattered light selecting optical device and the autofluorescence selecting optical device may be installed in parallel so that optical paths of the scattered light and the autofluorescence become separate systems. In this case, a short-pass filter which transmits only a light whose wavelength is shorter than the 410 nm cutoff wavelength is used as the scattered light selecting optical device. Then, the condensing lens optical systems which gather the scattered light and the autofluorescence 35e from the flow cell, and the scattered light receiving device photodiode 110 and the autofluorescence receiving device 90 are installed in front of and at the back of the respective optical devices. For example, the scattered light selecting optical device (short-pass filter) is installed at a 90 degree position (horizontal plane) from the optical axis of the laser light 31, and the autofluorescence selecting optical device (long-pass filter) is installed at a 90 degree position (vertical plane) from the optical axis of the laser light 31. Consequently, owing to the scattered light selecting optical device (short-pass filter), the scattered light receiving device photodiode 110 can detect mainly the scattered light from the viable particle 35 or the non-viable particle 37. Further, owing to the autofluorescence selecting optical device (long-pass filter), the autofluorescence receiving device photomultiplier 90 can detect mainly the autofluorescence 35e from the viable particle 35.

Further, by radiating the laser light 31 having a wavelength corresponding to an autofluorescent substance and providing the dichroic mirror which reflects the scattered light by the target and the long-pass filter which reduces the Raman-scattered light 33s by water or the like and transmits the autofluorescence 35e from the viable particle 35, it is possible to improve counting accuracy of the viable particles 35.

As described above, according to this embodiment, by using the viable counter 77, regarding the purified water stored in the purified water basin 818, it is possible to determine in real time whether or not the viable particle 35 exists. Incidentally, in the determination on the presence/absence of the viable particles regarding the purified water as well, the detection of the autofluorescence 35e is used as an index. Further, the autofluorescence 35e is emitted from the autofluorescent substance necessary for metabolism occurring in a living organism, such as riboflavin, NAD(P)H, or chlorophyll in the cell of the viable particle 35 being a detection (measurement) target. When as a result of the counting by the viable particle counter 77, it is confirmed that a prescribed number of the viable particles 35 or more exist in the purified water for which the counting is performed, the result is notified to the outside from a notifying means provided in the viable particle counter 77. For example, it is possible to output notifying sound from a speaker or output a notification signal for notifying the result to the central monitoring and controlling device 880. The central monitoring and control device 880 is capable of deciding a supply amount of the chlorine in real time based on the notification signal on the purified water, and is capable of controlling the regulating valves in the chemical mixture controlling device based on the decision contents. Therefore, when a prescribed number of the viable particles 35 or more exist in the purified water, it is possible to adjust a supply amount of the chlorine in real time.

Next, a purified water monitoring system, not part of the present invention, including the viable particle counter 77 will be described.

### [Purified water monitoring system including viable particle counter]

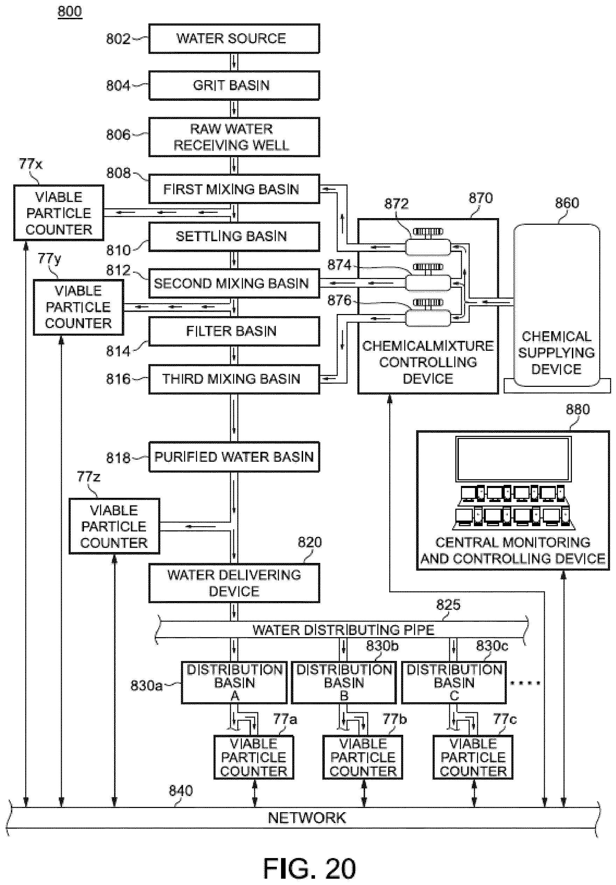

FIG. 20 is an explanatory diagram of the purified water monitoring system using the viable particle counter.

As illustrated in FIG. 20, the purified water monitoring system not only includes a viable particle counter 77z for inspecting the purified water stored in the purified water basin 818 but also performs the inspection of the chlorinated water by the viable particle counter 77. Concretely, in order to inspect the water chlorinated in the first mixing basin 808, a viable particle counter 77x is provided between the first mixing basin 808 and the settling basin 810. Further, similarly, in order to inspect the water chlorinated in the second mixing basin 812, a viable particle counter 77y is provided between the second mixing basin 812 and the third mixing basin 816. Incidentally, regarding the viable particle counter 77x and the viable particle counter 77y, the water sent from the first mixing basin 808 and the second mixing basin 812 is divided by the above-described dividing device (dividing means) (dividing step), and it is determined whether or not the viable particle exists in the divided water, and when the viable particle exists, the number of the viable particles is counted.

Further, in the purified water monitoring system, the target water is not limited to the water chlorinated by the water purifying system 800. After the manufactured purified water stored in the purified water basin is sent by the water pump 820 to be once stored in the distribution basins through the water distribution pipe 825, the determination on whether or not the viable particles exist is also performed for the purified water stored in the distribution basins. Hereinafter, this will be concretely described.

### [Distribution basins]

In the distribution basins, in order to adjust a distribution amount of the purified water from the water purifying system 800, the purified water is temporarily stored. Note that a plurality of the distribution basins are provided via the water distribution pipe 825 as illustrated in FIG. 20. For example, a distribution basin A 830a, a distribution basin B 830b, a distribution basin C 803c, and so on are provided. In the purified water monitoring system, a viable particle counter 77a is installed for the distribution basin A 830a, a viable particle counter 77b is installed for the distribution basin B 830b, and a viable particle counter 77c is installed for the distribution basin C 830c. Further, the purified waters stored in the respective distribution basins 830a, 830b, 830c are divided by dividing devices (dividing means) of the respective viable particle counters 77a, 77b, 77c (dividing step), and regarding the divided purified waters, it is determined whether or not the viable particles exist. When the viable particles exist as a result of this determination, the number of the viable particles is counted.

### [Network]

A network 840 connects the central monitoring and controlling device 880 with the viable particle counters 77x, 77y, 77z, 77a, 77b, 77c. By this network 840, the determination results on the presence/absence of the viable particles from the respective viable particle counters and count values of the viable particles are transmitted to the central monitoring and controlling device 880. Incidentally, the central monitoring and controlling device 880 may transmit control signals to the viable particle counters. Concretely, the central monitoring and controlling device 880 may transmit control signals for starting and finishing the inspection of the water at places where the viable particle counters are provided, and may inspect the water in real time by remote-controlling the viable particle counters. By thus monitoring in real time the water at the places where the viable particle counters are provided , it is possible to adjust an amount of the chlorine for the chlorination in real time. Incidentally, a control signal for controlling the adjustment of an amount of the chlorine for this chlorination is also transmitted from the central monitoring and controlling device 880 to the chemical mixture controlling device 870 via the network 840, so that the regulating valves 872, 874, 876 are adjusted. Here, when a problem is found in the purified water in or after the purified water basin 818, a regulating valve 878 is controlled via the network 840. Consequently, it is possible to inject the chlorine to a spare water distribution pipe for chlorination provided between the purified water basin 818 and the water pump 820.

As described above, being provided with the plural viable particle counters 77, the purified water monitoring system is capable of the real-time inspection not only of the purified water (purified water basin 818) manufactured by the water purifying system 800 but also of the waters in the plural water distribution basins. Concretely, the chlorinated water (the first mixing basin 808, the second mixing basin 812) can be inspected in real time. Further, based on the results of these inspections, the central monitoring and controlling device 880 decides an injection amount of the chlorine in the chlorination. Further, when the control signal based on the decided injection amount is transmitted from the central monitoring and controlling device 880, the regulating valves of the chemical mixture controlling device 870 are controlled based on the control signal. Therefore, it is possible to confirm an increase/decrease of seaweeds, microorganisms, and so on due to a climate change based on the inspection using the plural counting apparatuses 77, and it is possible to adjust the injection amount of the chlorine according to the inspection result. Further, it is possible to prevent too large an amount of the chlorine from being injected more than necessary. Accordingly, it is possible to reduce damage to the water distribution pipes and the generation of chlorination by-product (trihalomethane or the like) produced by the chlorination that affects a human body.

## Claims

1. A viable particle counter (77) for counting viable particles contained in a dialysis fluid, the viable particle counter (77) comprising:
a light emitting means (10) configured to radiate a light with a predetermined wavelength toward the dialysis fluid containing a detection target;
an autofluorescence selecting optical means (70) configured to reduce transmission of a Raman-scattered light emitted from the dialysis fluid out of lights emitted due to an interaction of the detection target or the dialysis fluid with the light radiated from the light emitting means (10) and to transmit an autofluorescence emitted from the detection target;
a viable particle determining means (2) configured to determine whether or not the detection target contained in the dialysis fluid is a viable particle, based on a light obtained after the Raman-scattered light is reduced by the autofluorescence selecting optical means (70); and
a liquid distributing means (720) configured to distribute the dialysis fluid for blood dialysis or blood filtration,
wherein the light emitting means (10) radiates the light with the predetermined wavelength that causes the autofluorescence and the Raman-scattered light which are emitted after the irradiation to be different in peak wavelength, and
wherein, based on the autofluorescence out of the lights emitted due to an interaction between the detection target contained in the dialysis fluid and the light radiated from the light emitting means (10), the viable particle determining means (2) determines whether or not the detection target contained in the dialysis fluid is the viable particle,
**characterized by**
a liquid dividing means (725) configured to make part of the dialysis fluid distributed by the liquid distributing means (720) branch off as the dialysis fluid containing the detection target before the dialysis fluid is administered to a human body,
wherein the light emitting means (10) radiates the light with the predetermined wavelength toward the dialysis fluid made to branch off by the liquid dividing means (725).

2. The viable particle counter (77) according to claim 1, further comprising:
a scattered light selecting optical means (60) configured to reflect a scattered light emitted from the detection target and to transmit the light including the autofluorescence and the Raman-scattered light,
wherein the viable particle determining means (2) determines whether or not the detection target contained in the dialysis fluid is the viable particle, based on the light transmitted by both the scattered light selecting optical means (60) and the autofluorescence selecting optical means (70).

3. The viable particle counter (77) according to claim 2, further comprising:
an autofluorescence receiving means (90) configured to receive the light transmitted by the autofluorescence selecting optical means (70) to output a first signal having an intensity corresponding to an amount of the received light;
a scattered light receiving means (110) configured to receive the light reflected by the scattered light selecting optical means (60) to output a second signal having an intensity corresponding to an amount of the received light;
a scattered light detection signal outputting means (300) configured to, when the intensity of the second signal output by the scattered light receiving means (110) is equal to or larger than a predetermined threshold value, determine that the scattered light emitted from the detection target contained in the dialysis fluid is detected and output a detection signal; and
a light shielding wall (65) configured to prevent light incident from a place other than an optical path from entering the optical path, which leads from the scattered light selecting optical means (60) to the autofluorescence receiving means (90) through the autofluorescence selecting optical means (70),
wherein, in the case where the detection signal is output by the scattered light detection signal outputting means (300), when the light is received by the autofluorescence receiving means (90) at the same time as an instant when the scattered light emitted from the detection target contained in the dialysis fluid is received by the scattered light receiving means (110) and the intensity of the first signal corresponding to the light received by the autofluorescence receiving means (90) at the same time as the instant is equal to or larger than a predetermined threshold value, the viable particle determining means (2) determines that the detection target contained in the dialysis fluid corresponding to the detection signal output by the scattered light detection signal outputting means (300) is the viable particle.

4. The viable particle counter (77) according to any one of claim 1 to claim 3,
wherein the predetermined wavelength of the light radiated from the light emitting means (10) is 375 nm to 420 nm, and
wherein a cutoff wavelength serving as the reference for the transmission of the light by the autofluorescence selecting optical means (70) is 450 nm to 490 nm.

5. A dialysis fluid monitoring system including the viable particle counter (77) according to any one of claim 1 to claim 4, the system comprising:
a dialysis fluid supplying means (630) for many people configured to supply the dialysis fluid for a plurality of people;
a dialysis monitoring device (710) for each patient configured to monitor and adjust the flow of the dialysis fluid for a patient;
a liquid distributing means (720m) for many people configured to divide and to distribute the dialysis fluid supplied by the dialysis fluid supplying means (630) for many people to each of the dialysis monitoring devices (710); and
a notifying means (76, 400, 640, 710) configured to notify a determination result by the viable particle counter (77),
wherein the viable particle counter (77) is provided in at least one of the dialysis fluid supplying means (630) for many people and the plural dialysis monitoring devices (710) and, regarding the dialysis fluid flowing in the dialysis fluid supplying means (630) for many people or the dialysis fluid divided to the dialysis monitoring devices (710), determines whether
or not the detection target contained in the dialysis fluid is a viable particle.

6. The dialysis fluid monitoring system according to claim 5,
wherein the viable particle counter (77) is provided in each of the dialysis monitoring devices (710).

## Patentansprüche

1. Lebendpartikelzähler (77) zum Zählen von Lebendpartikeln, die in einem Dialysefluid enthalten sind, wobei der Lebendpartikelzähler (77) Folgendes umfasst:
ein Lichtaussendemittel (10), das dafür ausgestaltet ist, ein Licht mit einer zuvor festgelegten Wellenlänge in Richtung des Dialysefluids, das ein Detektionsziel enthält, abzustrahlen;
ein optisches Eigenfluoreszenz-Auswahlmittel (70), das dafür ausgestaltet ist, das Durchlassen eines von dem Dialysefluid ausgesendeten Raman-gestreuten Lichts unter Lichtern zu reduzieren, die aufgrund einer Interaktion des Detektionsziels oder des Dialysefluids mit dem von dem Lichtaussendemittel (10) ausgesendeten Licht ausgesendet werden, und eine von dem Detektionsziel ausgesendete Eigenfluoreszenz durchzulassen;
eine Lebendpartikel-Bestimmungsvorrichtung (2), die dafür ausgestaltet ist, anhand eines Lichts, das erhalten wird, nachdem das Raman-gestreute Licht durch das optische Eigenfluoreszenz-Auswahlmittel (70) reduziert wurde, zu bestimmen, ob das in dem Dialysefluid enthaltene Detektionsziel ein Lebendpartikel ist oder nicht; und
ein Flüssigkeitsverteilungsmittel (720), das dafür ausgestaltet ist, das Dialysefluid zur Blutdialyse oder Blutfiltration zu verteilen,
wobei das Lichtaussendemittel (10) das Licht mit der zuvor festgelegten Wellenlänge abstrahlt, die bewirkt, dass die Eigenfluoreszenz und das Raman-gestreute Licht, die nach der Bestrahlung ausgesendet werden, unterschiedliche Spitzenwellenlängen haben, und
wobei die Lebendpartikel-Bestimmungsvorrichtung (2) anhand der Eigenfluoreszenz unter den Lichtern, die aufgrund einer Interaktion zwischen dem in dem Dialysefluid enthaltenen Detektionsziel und dem von dem Lichtaussendemittel (10) ausgesendeten Licht ausgesendet werden, bestimmt, ob das in dem Dialysefluid enthaltene Detektionsziel der Lebendpartikel ist oder nicht,
**gekennzeichnet durch**
ein Flüssigkeitsaufteilungsmittel (725), das dafür ausgestaltet ist zu veranlassen, dass ein Teil des durch das Flüssigkeitsverteilungsmittel (720) verteilten Dialysefluids als das Dialysefluid, welches das Detektionsziel enthält, abgezweigt wird, bevor das Dialysefluid in den Körper eines Menschen eingeleitet wird,
wobei das Lichtaussendemittel (10) das Licht mit der zuvor festgelegten Wellenlänge in Richtung des Dialysefluids abstrahlt, dessen Abzweigung durch das Flüssigkeitsaufteilungsmittel (725) veranlasst wurde.

2. Lebendpartikelzähler (77) nach Anspruch 1, des Weiteren umfassend:
ein optisches Streulicht-Auswahlmittel (60), das dafür ausgestaltet ist, ein von dem Detektionsziel ausgesendetes gestreutes Licht zu reflektieren und das Licht, einschließlich der Eigenfluoreszenz und des Raman-gestreuten Lichts, durchzulassen,
wobei die Lebendpartikel-Bestimmungsvorrichtung (2) anhand des Lichts, das sowohl durch das optische Streulicht-Auswahlmittel (60) als auch durch das optische Eigenfluoreszenz-Auswahlmittel (70) durchgelassen wird, bestimmt, ob das in dem Dialysefluid enthaltene Detektionsziel der Lebendpartikel ist oder nicht.

3. Lebendpartikelzähler (77) nach Anspruch 2, des Weiteren umfassend:
ein Eigenfluoreszenz-Empfangsmittel (90), das dafür ausgestaltet ist, das durch das optische Eigenfluoreszenz-Auswahlmittel (70) ausgesendete Licht zu empfangen, um ein erstes Signal auszugeben, das eine Intensität aufweist, die einem Betrag des empfangenen Lichts entspricht;
ein Streulicht-Empfangsmittel (110), das dafür ausgestaltet ist, das durch das optische Streulicht-Auswahlmittel (60) reflektierte Licht zu empfangen, um ein zweites Signal auszugeben, das eine Intensität aufweist, die einem Betrag des empfangenen Lichts entspricht;
ein Streulichtdetektionssignal-Ausgabemittel (300), das dafür ausgestaltet ist, wenn die Intensität des durch das Streulicht-Empfangsmittel (110) ausgegebenen zweiten Signals mindestens so groß ist wie ein zuvor festgelegter Schwellenwert, zu bestimmen, dass das gestreute Licht, das von dem in dem Dialysefluid enthaltenen Detektionsziel ausgesendet wird, detektiert wird, und ein Detektionssignal auszugeben; und
eine Lichtabschirmungswand (65), die dafür ausgestaltet ist zu verhindern, dass Licht, das von einer anderen Stelle als einem optischen Pfad her einfällt, in den optischen Pfad gelangt, der von dem optischen Streulicht-Auswahlmittel (60) durch das optische Eigenfluoreszenz-Auswahlmittel (70) hindurch zu dem Eigenfluoreszenz-Empfangsmittel (90) führt,
wobei für den Fall, dass das Detektionssignal durch das Streulichtdetektionssignal-Ausgabemittel (300) ausgegeben wird, wenn das Licht durch das Eigenfluoreszenz-Empfangsmittel (90) zur selben Zeit empfangen wird wie ein Moment, in dem das gestreute Licht, das von dem in dem Dialysefluid enthaltenen Detektionsziel ausgesendet wird, durch das Streulicht-Empfangsmittel (110) empfangen wird, und die Intensität des ersten Signals, die dem Licht entspricht, das durch das Eigenfluoreszenz-Empfangsmittel (90) zur selben Zeit empfangen wird wie der Moment, mindestens so groß ist wie ein zuvor festgelegter Schwellenwert, das Lebendpartikel-Bestimmungsvorrichtung (2) bestimmt, dass das in dem Dialysefluid enthaltene Detektionsziel, das dem durch das Streulichtdetektionssignal-Ausgabemittel (300) ausgegebenen Detektionssignal entspricht, der Lebendpartikel ist.

4. Lebendpartikelzähler (77) nach einem von Anspruch 1 bis Anspruch 3,
wobei die zuvor festgelegte Wellenlänge des von dem Lichtaussendemittel (10) abgestrahlten Lichts 375 nm bis 420 nm beträgt, und
wobei eine Grenzwellenlänge, die als die Referenz für das Durchlassen des Lichts durch das optische Eigenfluoreszenz-Auswahlmittel (70) dient, 450 nm bis 490 nm beträgt.

5. Dialysefluid-Überwachungssystem, das den Lebendpartikelzähler (77) nach einem von Anspruch 1 bis Anspruch 4 umfasst, wobei das System Folgendes umfasst:
ein Dialysefluid-Zufuhrmittel (630) für viele Personen, das dafür ausgestaltet ist, das Dialysefluid für mehrere Personen zuzuführen;
ein Dialyseüberwachungsgerät (710) für jeden Patienten, das dafür ausgestaltet ist, den Fluss des Dialysefluids für einen Patienten zu überwachen und einzustellen;
ein Flüssigkeitsverteilungsmittel (720m) für viele Personen, das dafür ausgestaltet ist, das durch das Dialysefluid-Zufuhrmittel (630) zugeführte Dialysefluid für viele Personen zu jedem der Dialyseüberwachungsgeräte (710) aufzuteilen und zu verteilen; und
ein Bekanntgabemittel (76, 400, 640, 710), das dafür ausgestaltet ist, ein Bestimmungsresultat durch den Lebendpartikelzähler (77) bekannt zu geben,
wobei der Lebendpartikelzähler (77) in mindestens einem des Dialysefluid-Zufuhrmittels (630) für viele Personen und den mehreren Dialyseüberwachungsgeräten (710) angeordnet ist und in Bezug auf das Dialysefluid, das in dem Dialysefluid-Zufuhrmittel (630) für viele Personen fließt, oder das Dialysefluid, das zu den Dialyseüberwachungsgeräten (710) aufteilt wird, bestimmt, ob das in dem Dialysefluid enthaltene Detektionsziel ein Lebendpartikel ist oder nicht.

6. Dialysefluid-Überwachungssystem nach Anspruch 5, wobei der Lebendpartikelzähler (77) in jedem der Dialyseüberwachungsgeräte (710) angeordnet ist.

## Revendications

1. Compteur de particules viables (77) destiné à compter des particules viables contenues dans un fluide dialyse, le compteur de particules viables (77) comprenant :
un moyen d'émission de lumière (10) conçu pour projeter une lumière d'une longueur d'onde prédéterminée, vers le fluide de dialyse contenant une cible de détection ;
un moyen optique de sélection d'autofluorescence (70) conçu pour réduire la transmission d'une lumière diffusée par effet Raman, émise à partir du fluide de dialyse,
parmi des lumières émises du fait d'une interaction de la cible de détection ou du fluide de dialyse avec la lumière projetée à partir du moyen d'émission de lumière (10) et pour transmettre une autofluorescence émise à partir de la cible de détection ;
un moyen de détermination de particules viables (2) conçu pour déterminer si la cible de détection contenue dans le fluide de dialyse est ou non une particule viable, en fonction d'une lumière obtenue après que la lumière diffusée par effet Raman a été réduite par le moyen optique de sélection d'autofluorescence (70) ; et
un moyen de distribution de liquide (720) conçu pour distribuer le fluide de dialyse à des fins de dialyse du sang ou de filtration du sang,
dans lequel le moyen d'émission de lumière (10) projette la lumière ayant la longueur d'onde prédéterminée qui fait que l'autofluorescence et la lumière diffusée par effet Raman qui sont émises après l'exposition au rayonnement diffèrent par leur pic de longueur d'onde, et
dans lequel, en fonction de l'autofluorescence parmi les lumières émises du fait d'une interaction entre la cible de détection contenue dans le fluide de dialyse et la lumière projetée à partir du moyen d'émission de lumière (10), le moyen de détermination de particules viables (2) détermine si la cible de détection contenue dans le fluide de dialyse est ou non la particule viable,
**caractérisé par** :
un moyen de division de liquide (725) conçu pour faire bifurquer une partie du fluide de dialyse distribué par le moyen de distribution de liquide (720), en tant que fluide de dialyse contenant la cible de détection, avant que le fluide de dialyse ne soit administré à un corps humain,
dans lequel le moyen d'émission de lumière (10) projette la lumière ayant la longueur d'onde prédéterminée vers le fluide de dialyse amené à bifurquer par le moyen de division de liquide (725).

2. Compteur de particules viables (77) selon la revendication 1, comprenant en outre :
un moyen optique de sélection de lumière diffusée (60) conçu pour réfléchir une lumière diffusée, émise à partir de la cible détection, et pour transmettre la lumière incluant l'autofluorescence et la lumière diffusée par effet Raman,
dans lequel le moyen de détermination de particules viables (2) détermine si la cible de détection contenue dans le fluide de dialyse est ou non la particule viable, en fonction de la lumière transmise à la fois par le moyen optique de sélection de lumière diffusée (60) et par le moyen optique de sélection d'autofluorescence (70).

3. Compteur de particules viables (77) selon la revendication 2, comprenant en outre :
un moyen de réception d'autofluorescence (90) conçu pour recevoir la lumière transmise par le moyen optique de sélection d'autofluorescence (70) afin de délivrer un premier signal ayant une intensité correspondant à une quantité de la lumière reçue ;
un moyen de réception de lumière diffusée (110) conçu pour recevoir la lumière réfléchie par le moyen optique de sélection lumière diffusée (60) afin de délivrer un second signal ayant une intensité correspondant à une quantité de la lumière reçue ;
un moyen émetteur de signaux de détection de lumière diffusée (300) conçu pour, lorsque l'intensité du second signal délivré par le moyen de réception de lumière diffusée (110) est supérieure ou égale à une valeur de seuil prédéterminée, déterminer que la lumière diffusée, émise à partir de la cible de détection contenue dans le fluide de dialyse est détectée et délivrer un signal de détection ; et
une paroi de protection contre la lumière (65) conçue pour empêcher une lumière incidente provenant d'un autre emplacement qu'un chemin optique de pénétrer dans le chemin optique qui mène du moyen optique de sélection de lumière diffusée (60) au moyen de réception d'autofluorescence (90) par le biais du moyen optique de sélection d'autofluorescence (70),
dans lequel, dans le cas où le signal de détection est délivré par le moyen émetteur de signaux de détection de lumière diffusée (300), lorsque la lumière est reçue par le moyen de réception d'autofluorescence (90) au même instant que celui où la lumière diffusée émise à partir de la cible de détection contenue dans le fluide de dialyse est reçue par le moyen de réception de lumière diffusée (110) et que l'intensité du premier signal correspondant à la lumière reçue par le moyen de réception d'autofluorescence (90) au même instant est supérieure ou égale à une valeur de seuil prédéterminée, le moyen de détermination de particules viables (2) détermine que la cible de détection contenue dans le fluide de dialyse correspondant au signal de détection délivré par le moyen émetteur de signaux de détection de lumière diffusée (300) est la particule viable.

4. Compteur de particules viables (77) selon l'une quelconque des revendications 1 à 3,
dans lequel la longueur d'onde prédéterminée de la lumière projetée à partir du moyen d'émission de lumière (10) est de 375 nm à 420 nm, et
dans lequel une longueur d'onde de coupure servant de référence pour la transmission de la lumière par le moyen optique de sélection d'autofluorescence (70) est de 450 nm à 490 nm.

5. Système de surveillance de fluide de dialyse comportant le compteur de particules viables (77) selon l'une quelconque des revendications 1 à 4, le système comprenant :
un moyen d'apport de fluide de dialyse (630) destiné à de nombreuses personnes, conçu pour fournir le fluide de dialyse à une pluralité de personnes ;
un moyen de surveillance de dialyse (710) destiné à chaque patient, conçu pour surveiller et ajuster le débit du fluide de dialyse destiné à un patient ;
un moyen de distribution de liquide (720m) destiné à de nombreuses personnes, conçu pour diviser et distribuer le fluide de dialyse fourni par le moyen d'apport de fluide de dialyse (630) destiné à de nombreuses personnes, à chacun des dispositifs de surveillance de dialyse (710) ; et
un moyen de notification (76, 400, 640, 710) conçu pour notifier un résultat de détermination par le compteur de particules viables (77),
dans lequel le compteur de particules viables (77) est mis en place dans le moyen d'apport de fluide de dialyse (630) destiné à de nombreuses personnes et/ou dans la pluralité de dispositifs de surveillance de dialyse (710) et, pour ce qui concerne le fluide de dialyse s'écoulant dans le moyen d'apport de fluide de dialyse (630) destiné à de nombreuses personnes ou le fluide de dialyse divisé à destination des dispositifs de surveillance de dialyse (710), détermine si la cible de détection contenue dans le fluide de dialyse est ou non une particule viable.

6. Système de surveillance de fluide de dialyse selon la revendication 5,
dans lequel le compteur de particules viables (77) est mis en place dans chacun des dispositifs de surveillance de dialyse (710).
